(19) Europäisches Patentamt
European Patent Office
Office européen des brevets

(11) EP 4 790 027 A1

(12) EUROPEAN PATENT APPLICATION
published in accordance with Art. 153(4) EPC

(43) Date of publication:
**12.08.2026 Bulletin 2026/33**

(21) Application number: **24885261.8**

(22) Date of filing: **26.08.2024**

(51) International Patent Classification (IPC):
***C02F 1/469*** *(2023.01)*

(52) Cooperative Patent Classification (CPC):
**C02F 1/469;** Y02A 20/124

(86) International application number:
**PCT/JP2024/030314**

(87) International publication number:
**WO 2025/094484 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 PCT/JP2023/039331**

(71) Applicant: **KIRIN HOLDINGS KABUSHIKI KAISHA**
**Nakano-ku, Tokyo 164-0001 (JP)**

(72) Inventors:
- **TANIGUCHI, Yoshimasa**
**Tokyo 164-0001 (JP)**
- **SAMPEI, Sotaro**
**Tokyo 164-0001 (JP)**
- **NARUSE, Tatsuro**
**Tokyo 164-0001 (JP)**
- **SASAKI, Toshinori**
**Tokyo 164-0001 (JP)**
- **TOKORO, Chiharu**
**Tokyo 169-8050 (JP)**
- **IWAI, Hisanori**
**Tokyo 169-8050 (JP)**

(74) Representative: **Hoffmann Eitle**
**Patent- und Rechtsanwälte PartmbB**
**Arabellastraße 30**
**81925 München (DE)**

# (54) METHOD FOR PRODUCING ORGANIC ACID

(57) An electrodialysis tank including an anode, a cathode, and one or more electrodialysis units is prepared, wherein each electrodialysis unit includes a bipolar membrane, a first cation exchange membrane, and a second cation exchange membrane, and a desalination chamber is formed between the first cation exchange membrane and the second cation exchange membrane. While applying a voltage between the anode and the cathode, an organic acid salt aqueous solution is circulated through a circulation path including the desalination chamber, thereby precipitating a solid containing a free organic acid in the desalination chamber. The method enables efficient production of an organic acid.

*Fig.2*

50
2
3a
3b
2a
20
30
40
11
13
NaOH
Na+
+
−
X-
H+
H+
OH-
XH
OH-
5

EP 4 790 027 A1

## Description

### Technical Field

The present disclosure relates to a method for producing or recovering, as a solid, a free organic acid by electrodialysis, and to a method for recycling polyester using the same.

### Background Art

As a method for recovering an acid component from an aqueous solution containing an inorganic acid salt or an organic acid salt, an electrodialysis technique using a bipolar membrane is known.

For example, Patent Document 1 discloses, as a method for recovering a terephthalic acid component and an alkali component from an alkaline processing waste liquid of polyester fibers, a method using an ion-exchange membrane electrodialysis tank partitioned by a bipolar membrane, an anion exchange membrane, and a cation exchange membrane to form an anode chamber, an acid component concentration chamber, a desalination chamber, an alkali component concentration chamber, and a cathode chamber, in which electrodialysis is performed by circulating a treated liquid of the alkaline processing waste liquid to the desalination chamber, thereby recovering the terephthalic acid component in the acid component concentration chamber and recovering the alkali component in the alkali concentration chamber and the cathode chamber. In addition, Non-Patent Document 1 discloses, as a method for recovering oily naphthenic acid, a method by electrodialysis using an apparatus including a combination of a bipolar membrane and two cation exchange membranes.

### Citation List

### Patent Literature

Patent Document 1: Japanese Unexamined Patent Application Publication No. H05-271150

### Non Patent Literature

Non-Patent Document 1: Separation and Purification Technology, 212 (2019), 929-940

### Summary of Invention

### Technical Problem

When a solid containing a free organic acid is recovered by the conventional method disclosed in Patent Document 1, an organic acid anion needs to permeate through an anion exchange membrane. However, the free organic acid may precipitate inside the anion exchange membrane, thereby clogging the membrane, and in order to prevent this, it has been necessary to always precisely control the pH of the acid component concentration chamber and the desalination chamber.

The present disclosure relates to a method for more efficiently recovering a solid containing a free organic acid from an aqueous solution containing an organic acid salt, or to a method for producing a solid containing a free organic acid.

### Solution to Problem

The present disclosure includes the following [1] to [13], [1'] to [11'], and [1"] to [10"].

[1] A method for producing an organic acid, comprising:

preparing an electrodialysis tank including an anode, a cathode, and one or more electrodialysis units provided between the anode and the cathode, wherein each of the electrodialysis units includes, sequentially arranged from the anode side toward the cathode side, a bipolar membrane, a first cation exchange membrane, and a second cation exchange membrane, an acid component chamber is formed between the bipolar membrane and the first cation exchange membrane, a desalination chamber is formed between the first cation exchange membrane and the second cation exchange membrane, and an alkali component chamber is formed on the cathode side of the second cation exchange membrane; and

while applying a voltage between the anode and the cathode, circulating an acid aqueous solution through a circulation path including the acid component chamber, circulating an alkali aqueous solution through a circulation path including the alkali component chamber, and circulating an organic acid salt aqueous solution containing an organic acid salt through a circulation path including the desalination chamber, thereby precipitating a solid containing a free organic acid generated from the organic acid salt in the desalination chamber.

[2] The method according to [1], wherein the solid contains crystals of the free organic acid.
[3] The method according to [2], wherein the crystals are needle-shaped crystals.
[4] The method according to any one of [1] to [3], wherein the organic acid salt aqueous solution further contains an inorganic salt.
[5] The method according to [4], wherein the inorganic salt includes one or more selected from the group consisting of sodium chloride, potassium chloride, sodium sulfate, potassium sulfate, sodium nitrate, and sodium dihydrogen phosphate.
[6] The method according to [4] or [5], wherein a concentration of the inorganic salt in the organic acid salt aqueous solution at 25°C before application of the voltage between the anode and the cathode is 0.001 M or more and 1.0 M or less.
[7] The method according to any one of [1] to [6], wherein the acid aqueous solution contains an acid, and a concentration of the acid in the acid aqueous solution at 25°C before application of the voltage between the anode and the cathode is 0.001 M or more and 2.0 M or less.
[8] The method according to any one of [1] to [7], wherein the organic acid salt is one or more selected from the group consisting of a carboxylate, a sulfonate, a sulfinate, an organic phosphate, and an organic phosphite.
[9] The method according to any one of [1] to [8], wherein the organic acid salt is a carboxylate.
[10] The method according to any one of [1] to [8], wherein the organic acid salt is a salt of a dicarboxylic acid.
[11] The method according to any one of [1] to [8], wherein the organic acid salt is a salt of terephthalic acid.
[12] The method according to any one of [1] to [11], further comprising recovering an alkali component from the alkali component chamber.
[13] A method for producing an organic acid or recovering an organic acid, comprising:

preparing an electrodialysis tank including an anode, a cathode, and one or more electrodialysis units provided between the anode and the cathode, wherein each of the electrodialysis units includes, sequentially arranged from the anode side toward the cathode side, a bipolar membrane, a first cation exchange membrane, and a second cation exchange membrane, an acid component chamber is formed between the bipolar membrane and the first cation exchange membrane, a desalination chamber is formed between the first cation exchange membrane and the second cation exchange membrane, and an alkali component chamber is formed on the cathode side of the second cation exchange membrane; and
while applying a voltage between the anode and the cathode, circulating an acid aqueous solution through a circulation path including the acid component chamber, circulating an alkali aqueous solution through a circulation path including the alkali component chamber, and circulating an organic acid salt aqueous solution containing an organic acid salt through a circulation path including the desalination chamber, thereby precipitating a solid containing a free organic acid generated from the organic acid salt in the desalination chamber,
wherein the voltage is 1 V or more and 300 V or less.

[1'] A method for recovering an organic acid, comprising:

preparing an electrodialysis tank including an anode, a cathode, and one or more electrodialysis units provided between the anode and the cathode, wherein each of the electrodialysis units includes, sequentially arranged from the anode side toward the cathode side, a bipolar membrane, a first cation exchange membrane, and a second cation exchange membrane, an acid component chamber is formed between the bipolar membrane and the first cation exchange membrane, a desalination chamber is formed between the first cation exchange membrane and the second cation exchange membrane, and an alkali component chamber is formed on the cathode side of the second cation exchange membrane; and
while applying a voltage between the anode and the cathode, circulating an acid aqueous solution through a circulation path including the acid component chamber, circulating an alkali aqueous solution through a circulation path including the alkali component chamber, and circulating an organic acid salt aqueous solution containing an organic acid salt through a circulation path including the desalination chamber, thereby precipitating a solid containing a free organic acid generated from the organic acid salt in the desalination chamber.

[2'] The method according to [1'], wherein the solid contains crystals of the free organic acid.
[3'] The method according to [2'], wherein the crystals are needle-shaped crystals.
[4'] The method according to any one of [1'] to [3'], wherein the organic acid salt aqueous solution further contains an inorganic salt.
[5'] The method according to [4'], wherein the inorganic salt includes one or more selected from the group consisting of sodium chloride, potassium chloride, sodium sulfate, potassium sulfate, sodium nitrate, and sodium dihydrogen phosphate.
[6'] The method according to [4'] or [5'], wherein a concentration of the inorganic salt in the organic acid salt aqueous solution at 25°C before application of the voltage between the anode and the cathode is 0.001 M or more and 1.0 M or less.
[7'] The method according to any one of [1'] to [6'], wherein the acid aqueous solution contains an acid, and a concentration of the acid in the acid aqueous solution at 25°C before application of the voltage between the anode and the cathode is 0.001 M or more and 2.0 M or less.
[8'] The method according to any one of [1'] to [7'], wherein the organic acid salt is one or more selected from the group consisting of a carboxylate, a sulfonate, a sulfinate, an organic phosphate, and an organic phosphite.
[9'] The method according to any one of [1'] to [8'], wherein the organic acid salt is a carboxylate.
[10'] The method according to any one of [1'] to [9'], wherein the organic acid salt is a salt of a dicarboxylic acid.
[11'] The method according to any one of [1'] to [10'], further comprising recovering an alkali component from the alkali component chamber.

[1"] A method for recovering a carboxylic acid, comprising:

preparing an electrodialysis tank including an anode, a cathode, and one or more electrodialysis units provided between the anode and the cathode, wherein each of the electrodialysis units includes, sequentially arranged from the anode side toward the cathode side, a bipolar membrane, a first cation exchange membrane, and a second cation exchange membrane, an acid component chamber is formed between the bipolar membrane and the first cation exchange membrane, a desalination chamber is formed between the first cation exchange membrane and the second cation exchange membrane, and an alkali component chamber is formed on the cathode side of the second cation exchange membrane; and
while applying a voltage between the anode and the cathode, circulating an acid aqueous solution through a circulation path including the acid component chamber, circulating an alkali aqueous solution through a circulation path including the alkali component chamber, and circulating a carboxylate aqueous solution containing a carboxylate through a circulation path including the desalination chamber, thereby precipitating a solid containing a free carboxylic acid generated from the carboxylate in the desalination chamber.

[2"] The method according to [1"], wherein the solid contains crystals of the free carboxylic acid.
[3"] The method according to [2"], wherein the crystals are needle-shaped crystals.
[4"] The method according to any one of [1"] to [3"], wherein the carboxylate aqueous solution further contains an inorganic salt.
[5"] The method according to [4"], wherein the inorganic salt includes one or more selected from the group consisting of sodium chloride, potassium chloride, sodium sulfate, potassium sulfate, sodium nitrate, and sodium dihydrogen phosphate.
[6"] The method according to [4"] or [5"], wherein a concentration of the inorganic salt in the carboxylate aqueous solution at 25°C before application of the voltage between the anode and the cathode is 0.001 M or more and 1.0 M or less.
[7"] The method according to any one of [1"] to [6"], wherein the carboxylate is an alkali metal salt or an alkaline earth metal salt of one or more dicarboxylic acids.
[8] The method according to any one of [1"] to [7"], further comprising recovering an alkali component from the alkali component chamber.
[9"] A method for recycling polyester, comprising:

forming a carboxylate aqueous solution containing an alkali metal salt of a dicarboxylic acid by depolymerization of polyester; and
recovering the dicarboxylic acid from the carboxylate aqueous solution by the method according to any one of [1"] to [8"].

[10"] The method according to any one of [1"] to [8"], wherein the acid aqueous solution contains an acid, and a concentration of the acid in the acid aqueous solution at 25°C before application of the voltage between the anode and the cathode is 0.001 M or more and 2.0 M or less.

## Advantageous Effects of Invention

According to the present disclosure, a solid containing a free organic acid (such as a carboxylic acid) can be produced or recovered more efficiently from an aqueous solution containing an organic acid salt (such as a carboxylate).

## Brief Description of Drawings

FIG. 1 is a schematic diagram showing an example of a recovery apparatus for recovering a solid containing an organic acid.
FIG. 2 is a schematic diagram showing an example of an electrodialysis action in an electrodialysis unit.
FIG. 3 is a scanning electron microscope image of an anion exchange membrane after electrodialysis in Comparative Example 1.
FIG. 4 is a scanning electron microscope image of an anion exchange membrane after electrodialysis in Comparative Example 1.
FIG. 5 shows infrared absorption spectra of an anion exchange membrane after electrodialysis in Comparative Example 1, free terephthalic acid, and disodium terephthalate, and a differential infrared absorption spectrum between a liquid-contact portion and a non-liquid-contact portion.
FIG. 6 is a scanning electron microscope image of a first cation exchange membrane (non-liquid-contact portion) after electrodialysis in Example 2.
FIG. 7 is a scanning electron microscope image of a first cation exchange membrane (liquid-contact portion) after electrodialysis in Example 2.
FIG. 8 shows infrared absorption spectra of a first cation exchange membrane after electrodialysis in Example 2, free terephthalic acid, and disodium terephthalate, and a differential infrared absorption spectrum between a liquid-contact portion and a non-liquid-contact portion.
FIG. 9 is a graph showing a relationship between pH and conductivity in a desalination chamber and energization time in an electrodialysis test of Example 1.
FIG. 10 is a graph showing a relationship between pH and conductivity in a desalination chamber and energization time in an electrodialysis test of Example 2.
FIG. 11 is a graph showing a relationship between a remaining ratio of disodium terephthalate and energization time in electrodialysis tests of Examples 1 and 2.
FIG. 12 is a graph showing a relationship between a current flowing through an electrodialysis apparatus and energization time in electrodialysis tests of Examples 1 and 2.
FIG. 13 is a scanning electron microscope image of crystals of terephthalic acid recovered in an electrodialysis test of Example 2.
FIG. 14 is a graph showing a relationship between pH in a desalination chamber and energization time in an electrodialysis test of Example 3.
FIG. 15 is a graph showing a relationship between conductivity in a desalination chamber and energization time in an electrodialysis test of Example 3.
FIG. 16 is a graph showing a relationship between a remaining ratio of disodium terephthalate and energization time in an electrodialysis test of Example 3.
FIG. 17 is a graph showing a relationship between a current flowing through an electrodialysis apparatus and energization time in an electrodialysis test of Example 3.
FIG. 18 is a graph showing a relationship between pH in a desalination chamber and energization time in an electrodialysis test of Example 4.
FIG. 19 is a graph showing a relationship between conductivity in a desalination chamber and energization time in an electrodialysis test of Example 4.
FIG. 20 is a graph showing a relationship between a remaining ratio of disodium terephthalate and energization time in an electrodialysis test of Example 4.
FIG. 21 is a graph showing a relationship between a current flowing through an electrodialysis apparatus and energization time in an electrodialysis test of Example 4.
FIG. 22 is a schematic diagram showing an example of a recovery apparatus for electrodialysis test in Comparative Example 1.

FIG. 23 is a graph showing a relationship between pH in a desalination chamber and energization time in electrodialysis tests of Examples 9-11.

FIG. 24 is a graph showing a relationship between conductivity in a desalination chamber and energization time in electrodialysis tests of Examples 9-11.

FIG. 25 is a graph showing a relationship between a remaining ratio of disodium terephthalate and energization time in electrodialysis tests of Examples 9-11.

FIG. 26 is a graph showing a relationship between a current flowing through an electrodialysis apparatus and energization time in electrodialysis tests of Examples 9-11.

FIG. 27 is a graph showing a relationship between pH in a desalination chamber and energization time in electrodialysis tests of Examples 12-14.

FIG. 28 is a graph showing a relationship between conductivity in a desalination chamber and energization time in electrodialysis tests of Examples 12-14.

FIG. 29 is a graph showing a relationship between a remaining ratio of disodium terephthalate and energization time in electrodialysis tests of Examples 12-14.

FIG. 30 is a graph showing a relationship between a current flowing through an electrodialysis apparatus and energization time in an electrodialysis tests of Examples 12-14.

FIG. 31 is a graph showing a relationship between pH in a desalination chamber and energization time in electrodialysis tests of Examples 15-17.

FIG. 32 is a graph showing a relationship between conductivity in a desalination chamber and energization time in electrodialysis tests of Examples 15-17.

FIG. 33 is a graph showing a relationship between a remaining ratio of disodium terephthalate and energization time in electrodialysis tests of Examples 15-17.

FIG. 34 is a graph showing a relationship between a current flowing through an electrodialysis apparatus and energization time in an electrodialysis tests of Examples 15-17.

FIG. 35 is a graph showing a relationship between pH in a desalination chamber and energization time in electrodialysis tests of Examples 16, 18 and 19.

FIG. 36 is a graph showing a relationship between conductivity in a desalination chamber and energization time in electrodialysis tests of Examples 16, 18 and 19.

FIG. 37 is a graph showing a relationship between a remaining ratio of disodium terephthalate and energization time in electrodialysis tests of Examples 16, 18 and 19.

FIG. 38 is a graph showing a relationship between a current flowing through an electrodialysis apparatus and energization time in an electrodialysis tests of Examples 16, 18 and 19.

FIG. 39 is a graph showing a relationship between pH in a desalination chamber and energization time in electrodialysis tests of Examples 16, 20 and 21.

FIG. 40 is a graph showing a relationship between conductivity in a desalination chamber and energization time in electrodialysis tests of Examples 16, 20 and 21.

FIG. 41 is a graph showing a relationship between a remaining ratio of disodium terephthalate and energization time in electrodialysis tests of Examples 16, 20 and 21.

FIG. 42 is a graph showing a relationship between a current flowing through an electrodialysis apparatus and energization time in electrodialysis tests of Examples 16, 20 and 21.

FIG. 43 is a graph showing a relationship between pH in a desalination chamber and energization time in an electrodialysis test of Example 22.

FIG. 44 is a graph showing a relationship between conductivity in a desalination chamber and energization time in an electrodialysis test of Example 22.

FIG. 45 is a graph showing a relationship between a remaining ratio of disodium terephthalate and energization time in an electrodialysis test of Example 22.

FIG. 46 is a graph showing a relationship between a current flowing through an electrodialysis apparatus and energization time in an electrodialysis test of Example 22.

FIG. 47 is a graph showing a relationship between pH in a desalination chamber and energization time in an electrodialysis test of Example 23.

FIG. 48 is a graph showing a relationship between conductivity in a desalination chamber and energization time in an electrodialysis test of Example 23.

FIG. 49 is a graph showing a relationship between a remaining ratio of disodium terephthalate and energization time in an electrodialysis test of Example 23.

FIG. 50 is a graph showing a relationship between a current flowing through an electrodialysis apparatus and energization time in an electrodialysis test of Example 23.

FIG. 51 is a graph showing a relationship between pH in a desalination chamber and energization time in an electrodialysis test of Example 24.

FIG. 52 is a graph showing a relationship between conductivity in a desalination chamber and energization time in an electrodialysis test of Example 24.

FIG. 53 is a graph showing a relationship between a remaining ratio of disodium terephthalate and energization time in an electrodialysis test of Example 24.

FIG. 54 is a graph showing a relationship between a current flowing through an electrodialysis apparatus and energization time in an electrodialysis test of Example 24.

**Description of Embodiments**

The present invention is not limited to the following examples.

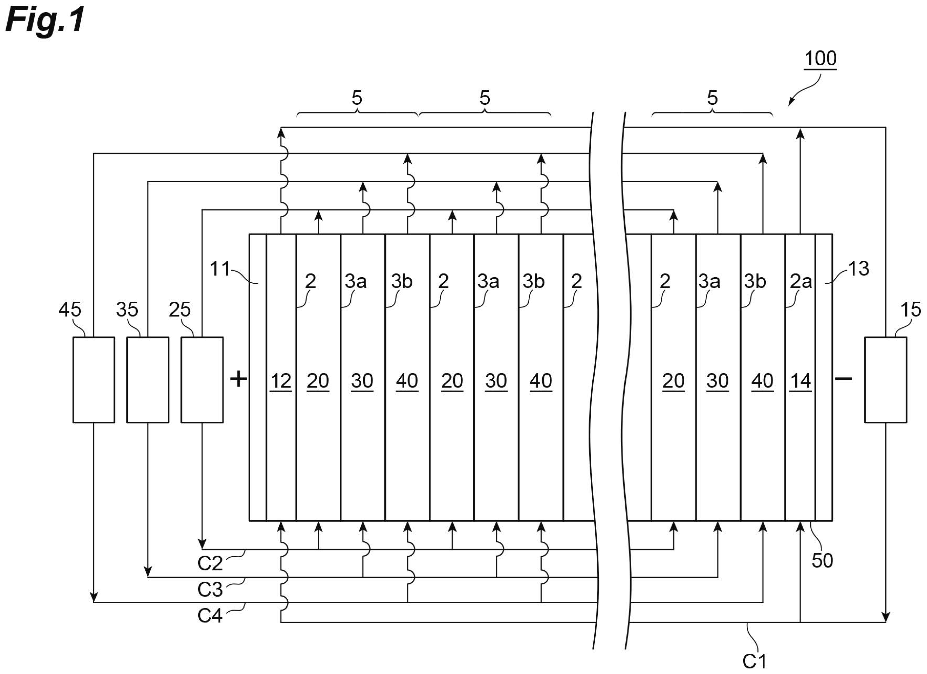

FIG. 1 is a schematic diagram showing an example of a recovery apparatus used for recovering a solid containing an organic acid by electrodialysis. A recovery apparatus 100 shown in FIG. 1 includes an electrodialysis tank 50, an electrolyte tank 15, an acid component chamber tank 25, a desalination chamber tank 35, an alkali component chamber tank 45, and piping connecting the electrodialysis tank 50 with the electrolyte tank 15, the acid component chamber tank 25, the desalination chamber tank 35, or the alkali component chamber tank 45. The electrodialysis tank 50 includes an anode chamber 12 including an anode 11, a cathode chamber 14 including a cathode 13, and a plurality of electrodialysis units 5 provided between the anode 11 (the anode chamber 12) and the cathode 13 (the cathode chamber 14). The plurality of electrodialysis units 5 are arranged in series from the anode 11 side toward the cathode 13 side. Each electrodialysis unit 5 includes, sequentially arranged from the anode 11 side toward the cathode 13 side, a bipolar membrane 2, a first cation exchange membrane 3a, and a second cation exchange membrane 3b. The bipolar membrane 2, the first cation exchange membrane 3a, and the second cation exchange membrane 3b are arranged with spaces therebetween. An acid component chamber 20 is formed between the bipolar membrane 2 and the first cation exchange membrane 3a, a desalination chamber 30 is formed between the first cation exchange membrane 3a and the second cation exchange membrane 3b, and an alkali component chamber 40 is formed on the cathode 13 side of the second cation exchange membrane 3b. The alkali component chamber 40 formed by each electrodialysis unit 5 is formed between the bipolar membrane 2 and the second cation exchange membrane 3b of an electrodialysis unit 5 positioned adjacent on the cathode 13 side. An additional bipolar membrane 2a is provided between the alkali component chamber 40 of the electrodialysis unit 5 positioned most on the cathode 13 side and the cathode chamber 14.

One electrodialysis unit 5 is a combination of one bipolar membrane 2, one first cation exchange membrane 3a, and one second cation exchange membrane 3b arranged via the acid component chamber 20 or the desalination chamber 30. The number of electrodialysis units included in one recovery apparatus can be determined such that a total area of the bipolar membranes 2, the first cation exchange membranes 3a, and the second cation exchange membranes 3b included in the recovery apparatus falls within a range set in consideration of processing capacity and the like of the recovery apparatus. The number of electrodialysis units 5 included in one electrodialysis tank 50 is not particularly limited, and may be, for example, 1 or more and 100 or less, 1 or more and 10 or less, or 10 or more and 100 or less.

An electrolyte aqueous solution is stored in the electrolyte tank 15, an acid aqueous solution is stored in the acid component chamber tank 25, an organic acid salt aqueous solution is stored in the desalination chamber tank 35, and an alkali aqueous solution is stored in the alkali component chamber tank 45. A circulation path C1 for circulating the electrolyte aqueous solution is formed by the anode chamber 12 and the cathode chamber 14, the electrolyte tank 15, and piping connecting these components. A circulation path C2 for circulating the acid aqueous solution is formed by the acid component chamber 20, the acid component chamber tank 25, and piping connecting these components. A circulation path C3 for circulating an organic acid salt aqueous solution containing an organic acid salt is formed by the desalination chamber 30, the desalination chamber tank 35, and piping connecting these components. A circulation path C4 for circulating the alkali aqueous solution is formed by the alkali component chamber 40, the alkali component chamber tank 45, and piping connecting these components. A liquid-feeding device such as a liquid-feeding pump is usually provided on each circulation path.

The anode 11, the cathode 13, the bipolar membrane 2, the first cation exchange membrane 3a, and the second cation exchange membrane 3b may be those commonly used in electrodialysis. The first cation exchange membrane 3a and the second cation exchange membrane 3b may be the same as or different from each other. The additional bipolar membrane 2a may be the same as the bipolar membrane 2. The area (effective area) of each of the bipolar membrane 2, the first cation exchange membrane 3a, and the second cation exchange membrane 3b is not particularly limited, and may be, for example, 20 $cm^2$ or more and 4 $m^2$ or less. The area herein refers to an area of each ion exchange membrane as viewed in a thickness direction. The volumes of the acid component chamber 20, the desalination chamber 30, and the alkali component chamber 40 are not particularly limited, and may be, for example, 2 $cm^3$ or more and 8 $m^3$ or less. The thicknesses of the bipolar membrane 2, the first cation exchange membrane 3a, and the second cation exchange membrane 3b are not particularly limited, and may be, for example, 0.15 mm or more and 0.30 mm or less. The distance between the bipolar membrane 2 and the first cation exchange membrane 3a (a width of the acid component chamber 20) is not particularly limited, and may be, for example, 1.0 mm or more and 30.0 mm or less. The distance between the first

cation exchange membrane 3a and the second cation exchange membrane 3b (a width of the desalination chamber 30) is not particularly limited, and may be, for example, 1.0 mm or more and 30.0 mm or less. The distance between the second cation exchange membrane 3b and the bipolar membrane 2 (a width of the alkali component chamber) is not particularly limited, and may be, for example, 1.0 mm or more and 30.0 mm or less. The widths of the anode chamber 12 and the cathode chamber 14 are not particularly limited, and may be, for example, 1.0 mm or more and 30.0 mm or less.

By electrodialysis in which an electrolyte aqueous solution is circulated through the circulation path C1, an acid aqueous solution is circulated through the circulation path C2, an alkali aqueous solution is circulated through the circulation path C4, and an organic acid salt aqueous solution is circulated through the circulation path C3 while applying a voltage between the anode 11 and the cathode 13, a solid containing a free organic acid generated from the organic acid salt is precipitated in the desalination chamber 30.

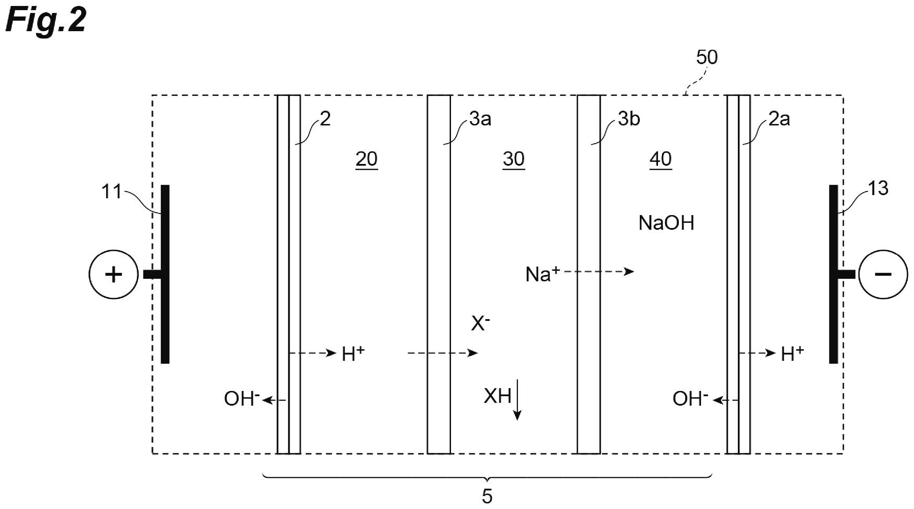

FIG. 2 is a schematic diagram showing an example of an electrodialysis action in an electrodialysis unit. FIG. 2 shows an example of electrodialysis in which a solid containing a free organic acid XH is recovered from an organic acid salt aqueous solution containing a sodium salt of an organic acid $X\text{-}Na^+$. When a voltage is applied between the anode 11 and the cathode 13, protons $H^+$ generated by dissociation of water from the bipolar membrane 2 are supplied to the acid component chamber 20, and hydroxide ions $OH^-$ generated by dissociation of water from the bipolar membrane 2a are supplied to the alkali component chamber 40. At the same time, the protons $H^+$ in the acid component chamber 20 permeate through the first cation exchange membrane 3a and move into the desalination chamber 30, and sodium cations $Na^+$ in the desalination chamber 30 permeate through the second cation exchange membrane 3b and move into the alkali component chamber 40. As a result, a solid containing a free organic acid XH exceeding a saturated concentration is precipitated in the desalination chamber 30. As the solubility of the free organic acid in water becomes lower, the free organic acid can be more efficiently recovered or produced as a solid. Since the organic acid anion $X^-$ does not substantially permeate through the first cation exchange membrane 3a, clogging of the membrane due to precipitation of the free organic acid within the membrane is suppressed. In addition, an alkali component (for example, sodium hydroxide (NaOH)) concentrated in the alkali component chamber 40 can also be produced or recovered. According to the method of the present disclosure, the alkali component can also be efficiently produced or recovered.

The solid precipitated in the desalination chamber 30 may be a substance that is solid at 25°C. The solid may contain crystals of the free organic acid. According to the method of the present disclosure, solid particles (or crystal particles) containing crystals of the organic acid and having a relatively large particle size can be efficiently produced. A large particle size is advantageous in terms of easy treatment of waste liquid generated in association with washing of the recovered solid. In addition, a large particle size is advantageous in terms of cost reduction of a production process, such as improvement in production efficiency due to shortening of filtration time and drying time during recovery and washing. For example, the precipitated solid may contain particles (particularly crystal particles) having a major axis length of 0.0001 mm or more. An upper limit of the major axis length of the particles is not particularly limited, and may be about 1 mm. The major axis length of the particles can be observed, for example, by a scanning electron microscope (SEM). Here, the major axis length refers to, for example, a maximum distance between two parallel straight lines sandwiching each particle in an SEM image. Particles having a large length may be needle-shaped crystals. The needle-shaped crystals may be formed, for example, of terephthalic acid. In addition, the particle size may also be quantitatively measured using a laser diffraction particle size distribution analyzer or the like. A D80 median diameter, represented by a cumulative distribution of particles containing crystals of the free organic acid, may be, for example, 10 μm or more and 1000 μm or less, or 100 μm or more and 1000 μm or less.

The organic acid salt contained in the organic acid salt aqueous solution supplied to the desalination chamber 30 is water-soluble. The organic acid salt may be a salt that forms a free organic acid having low water solubility. According to findings of the present inventors, even when a free organic acid having low water solubility is precipitated in the desalination chamber 30, highly efficient electrodialysis can be continuously performed for a long period of time. The organic acid constituting the organic acid salt may be a compound capable of forming an ester compound or a polyester by condensation or polycondensation with an alcohol compound (for example, a diol). The organic acid is a compound having an acidic functional group, and the number and type of acidic functional groups contained in one molecule are not limited. The acidic functional group of the organic acid may be, for example, a carboxyl group, a phenolic group, a sulfuric acid group, a sulfo group, a sulfino group, a phosphoric acid group, a phosphoryl group, or a phosphorous acid group.

Examples of the organic acid are not particularly limited, and include carboxylic acids, sulfonic acids, sulfinic acids, organic phosphoric acids, and organic phosphinic acids. That is, the organic acid salt may be one or more selected from the group consisting of carboxylates, sulfonates, sulfinates, organic phosphates, and organic phosphites, and may be a carboxylate. In particular, the organic acid may be a dicarboxylic acid.

Examples of monocarboxylic acids constituting the carboxylate are not particularly limited, and include benzoic acid, salicylic acid, ferulic acid, prostaglandin E1, and prostaglandin E2.

Examples of dicarboxylic acids constituting the carboxylate are not particularly limited, and include malonic acid, succinic acid, glutaric acid, adipic acid, suberic acid, sebacic acid, dodecanedioic acid, eicosanedioic acid, pimelic acid, azelaic acid, methylmalonic acid, ethylmalonic acid, adamantanedicarboxylic acid, norbomenedicarboxylic acid, cyclo-

hexanedicarboxylic acid, decalinedicarboxylic acid, terephthalic acid, isophthalic acid, phthalic acid, 1,4-naphthalenedicarboxylic acid, 1,5-naphthalenedicarboxylic acid, 2,6-naphthalenedicarboxylic acid, 1,8-naphthalenedicarboxylic acid, 4,4'-diphenyldicarboxylic acid, 4,4'-diphenyl ether dicarboxylic acid, 5-sodium sulfoisophthalic acid, phenylendanedicarboxylic acid, anthracenedicarboxylic acid, phenanthrenedicarboxylic acid, 9,9'-bis(4-carboxyphenyl)fluorene acid, and 2,5-furandicarboxylic acid.

Examples of tricarboxylic acids constituting the carboxylate are not particularly limited, and include trimesic acid and agaric acid.

Examples of sulfonic acids constituting the sulfonate are not particularly limited, and include benzenesulfonic acid.

Examples of sulfinic acids constituting the sulfinate are not particularly limited, and include benzenesulfinic acid.

Examples of organic phosphoric acids constituting the organic phosphate are not particularly limited, and include benzenephosphonic acid. Examples of organic phosphorous acids constituting the organic phosphite are not particularly limited, and include benzenephosphinic acid.

The organic acid salts such as carboxylates, sulfonates, sulfinates, organic phosphates, and organic phosphites may be alkali metal salts, alkaline earth metal salts, ammonium salts, or combinations thereof. The alkali metal salts may be one or more selected from lithium salts, sodium salts, potassium salts, rubidium salts, cesium salts, and francium salts. The alkaline earth metal salts may be one or more selected from beryllium salts, magnesium salts, calcium salts, strontium salts, barium salts, and radium salts. The organic acid salts (for example, carboxylates) may be, in particular, sodium salts, potassium salts, ammonium salts, or combinations thereof.

Specific examples of carboxylates include sodium benzoate, potassium benzoate, ammonium benzoate, magnesium benzoate, calcium benzoate, sodium salicylate, potassium salicylate, ammonium salicylate, magnesium salicylate, calcium salicylate, disodium terephthalate, sodium hydrogen terephthalate, dipotassium terephthalate, potassium hydrogen terephthalate, ammonium hydrogen terephthalate, diammonium terephthalate, magnesium terephthalate, calcium terephthalate, disodium 2,5-furandicarboxylate, sodium hydrogen 2,5-furandicarboxylate, dipotassium 2,5-furandicarboxylate, potassium hydrogen 2,5-furandicarboxylate, ammonium hydrogen 2,5-furandicarboxylate, diammonium 2,5-furandicarboxylate, magnesium 2,5-furandicarboxylate, and calcium 2,5-furandicarboxylate.

Specific examples of sulfonates include sodium benzenesulfonate, potassium benzenesulfonate, ammonium benzenesulfonate, magnesium benzenesulfonate, and calcium benzenesulfonate.

Specific examples of sulfinates include sodium benzenesulfinate, potassium benzenesulfinate, ammonium benzenesulfinate, magnesium benzenesulfinate, and calcium benzenesulfinate.

Specific examples of organic phosphates include sodium benzenephosphonate, potassium benzenephosphonate, ammonium benzenephosphonate, magnesium benzenephosphonate, and calcium benzenephosphonate.

Specific examples of organic phosphites include sodium benzenephosphinate, potassium benzenephosphinate, ammonium benzenephosphinate, magnesium benzenephosphinate, and calcium benzenephosphinate.

A concentration of the organic acid salt in the aqueous solution of the organic acid salt (for example, a carboxylate, a sulfonate, a sulfinate, an organic phosphate, or an organic phosphite to be subjected to electrodialysis can be arbitrarily adjusted within a range of a saturated concentration or less. A molar concentration of the organic acid salt in the organic acid salt aqueous solution at 25°C before initiation of electrodialysis may be, for example, 0.001 M or more, and may be 1.0 M or less, 0.90 M or less, 0.80 M or less, 0.70 M or less, 0.60 M or less, 0.50 M or less, 0.40 M or less, 0.30 M or less, 0.20 M or less, 0.10 M or less, 0.09 M or less, 0.08 M or less, 0.07 M or less, 0.06 M or less, 0.05 M or less, 0.04 M or less, or 0.03 M or less. One molar (1 M) corresponds to $1 \times 10^3$ mol/m$^3$. The molar concentration of the organic acid salt in the organic acid salt aqueous solution at 25°C before initiation of electrodialysis may be 0.001 M or more and 1.0 M or less.

The organic acid salt aqueous solution may further contain other components in addition to water and the organic acid salt. Examples of other components that may be contained in the organic acid salt aqueous solution include inorganic salts as electrolytes, alkali components (for example, sodium hydroxide), and alcohol compounds (for example, methanol, ethanol, and ethylene glycol).

The inorganic salt may be a compound that exhibits strong acidity in the presence of protons, and such a compound may be referred to as a conductor. When the organic acid salt aqueous solution contains such an inorganic salt, a decrease in pH of the organic acid salt aqueous solution during electrodialysis is promoted, thereby enhancing a conversion efficiency from the organic acid salt to the free organic acid. That is, conversion from the organic acid salt to the free organic acid can be completed in a shorter period of time. The inorganic salt that may be added to the organic acid salt aqueous solution may be, for example, a chloride, a sulfate, a nitrate, a phosphate, or a combination thereof. In particular, the inorganic salt may be one or more selected from sodium chloride, potassium chloride, sodium sulfate, potassium sulfate, sodium nitrate, and sodium dihydrogen phosphate. A concentration of the inorganic salt in the organic acid salt aqueous solution at 25°C before initiation of electrodialysis may be, for example, 0.0005 M or more, 0.005 M or more, 0.01 M or more, 0.02 M or more, 0.03 M or more, 0.04 M or more, 0.05 M or more, 0.06 M or more, 0.07 M or more, 0.08 M or more, or 0.09 M or more, and may be 1.0 M or less, 0.90 M or less, 0.80 M or less, 0.70 M or less, 0.60 M or less, 0.50 M or less, 0.40 M or less, 0.30 M or less, 0.20 M or less, or 0.10 M or less. The concentration of the inorganic salt in the organic

acid salt aqueous solution at 25°C before initiation of electrodialysis may be 0.0005 M or more and 1.0 M or less.

The electrolyte aqueous solution circulating through the circulation path C1 including the anode chamber 12 and the cathode chamber 14 may be an aqueous solution containing water and an electrolyte. The electrolyte is not particularly limited, and may be, for example, an inorganic alkali component (for example, sodium hydroxide or potassium hydroxide) or an inorganic salt.

The acid aqueous solution circulating through the circulation path C2 including the acid component chamber 20 may be an acidic aqueous solution containing water and an acid. The acid contained in the acid aqueous solution may be an inorganic acid (for example, sulfuric acid), an organic acid (for example, methanesulfonic acid), or a combination thereof. A concentration of the acid in the acid aqueous solution at 25°C before initiation of electrodialysis may be, for example, 0.001 M or more and 10.0 M or less, or 0.001 M or more and 2.0 M or less. The concentration of the acid in the acid aqueous solution at 25°C before initiation of electrodialysis may be 0.005 M or more, 0.05 M or more, or 0.07 M or more, and may be 1.4 M or less or 1.2 M or less.

The alkali aqueous solution circulating through the circulation path C4 including the alkali component chamber 40 may be an aqueous solution containing water and an alkali component (for example, sodium hydroxide). A concentration of the alkali component in the alkali aqueous solution at 25°C before initiation of electrodialysis may be, for example, 0.001 M or more and 10.0 M or less, or 0.001 M or more and 2.0 M or less.

During electrodialysis, a temperature of the electrolyte aqueous solution, the acid aqueous solution, the organic acid salt aqueous solution, and the alkali aqueous solution may be, for example, 0°C or more and 95°C or less, or 5°C or more and 70°C or less. A voltage applied between the anode 11 and the cathode 13 is not particularly limited, and may be, for example, 1 V or more and 300 V or less, 10 V or more and 300 V or less, 15 V or more and 300 V or less, or 30 V or more and 300 V or less.

The organic acid salt aqueous solution treated by the methods exemplified above may be an aqueous solution formed by depolymerization of polyester or the like. In such a case, polyester or the like can be efficiently recycled by a method including forming an organic acid salt aqueous solution containing an organic acid salt (for example, a dicarboxylate) by depolymerization of polyester or the like, and producing or recovering an organic acid (for example, a dicarboxylic acid) from the organic acid salt aqueous solution by the method according to the present disclosure. Examples of polyester to be recycled include polycarboxylic acid esters, polysulfuric acid esters, and polyphosphoric acid esters. The method according to the present disclosure may be applied to recycling of polyethylene terephthalate.

## EXAMPLES

The present invention is not limited to the following examples.

### 1. Apparatus and Materials

The following electrodialysis apparatus and ion exchange membranes (effective membrane area: 550 $cm^2$) were used.

Electrodialysis apparatus:
Ashlizer EX3B (trade name, manufactured by Astom Corporation)
Bipolar membrane:
Neosepta BPU (trade name, effective membrane area: 550 $cm^2$, manufactured by Astom Corporation)
Cation exchange membrane:
Neosepta CBM (trade name, effective membrane area: 550 $cm^2$, manufactured by Astom Corporation)
Anion exchange membrane:
Neosepta AHA (trade name, effective membrane area: 550 $cm^2$, manufactured by Astom Corporation)

### 2. Electrodialysis Test

#### Comparative Example 1

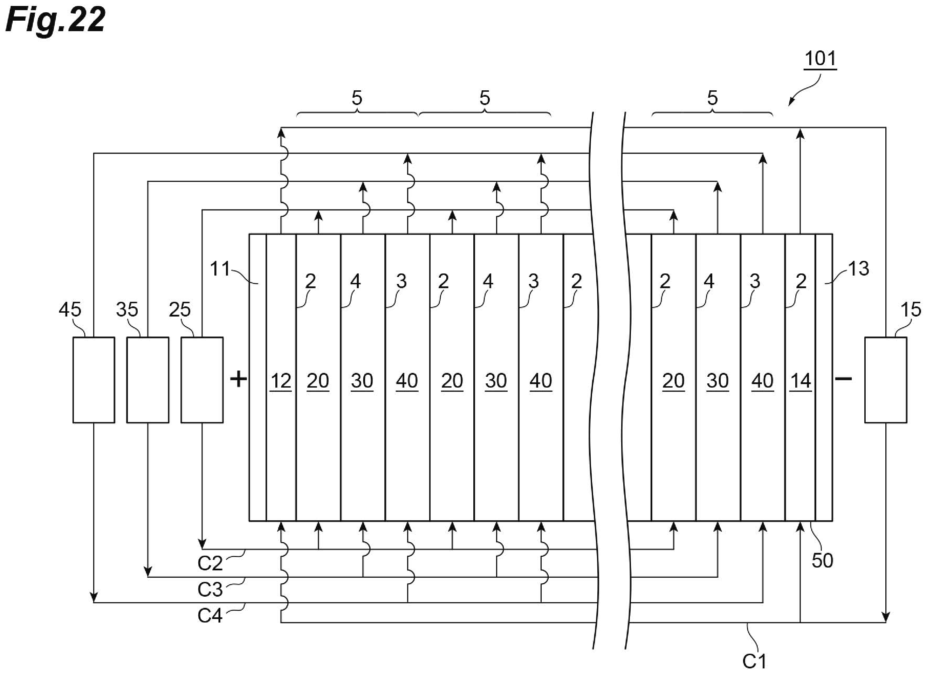

An electrodialysis apparatus having a configuration shown in FIG. 22 was prepared as a recovery apparatus for an organic acid. In a recovery apparatus 101 shown in FIG. 22, ten sets of ion exchange membranes, each set consisting of a bipolar membrane 2, an anion exchange membrane 4, and a cation exchange membrane 3, were sequentially arranged from the anode 11 side toward the cathode 13 side between an anode chamber 12 provided with an anode 11 and a cathode chamber 14 provided with a cathode 13. A bipolar membrane 2 was also arranged between the cation exchange membrane 3 positioned most on the cathode 13 side and the cathode 13. An acid component chamber 20 was formed between the bipolar membrane 2 and the anion exchange membrane 4, a desalination chamber 30 was formed between

the anion exchange membrane 4 and the cation exchange membrane 3, and an alkali component chamber 40 was formed between the cation exchange membrane 3 and the bipolar membrane 2 positioned on the cathode 13 side. Ten electrodialysis units 5, each composed of three chambers including the acid component chamber 20, the desalination chamber 30, and the alkali component chamber 40, were arranged in series between the anode 11 and the cathode 13.

An NaOH aqueous solution having a concentration of 0.5 M (1000 mL) was circulated as an electrolyte aqueous solution through a circulation path C1 including the anode chamber 12 and the cathode chamber 14. An $H_2SO_4$ aqueous solution having a concentration of 0.1 M (500 mL) was circulated through a circulation path C2 including the acid component chamber 20. An NaOH aqueous solution having a concentration of 0.1 M (500 mL) was circulated through a circulation path C4 including the alkali component chamber 40. A disodium terephthalate aqueous solution having a concentration of 0.5 M (500 mL), containing 10 volume% methanol, 0.5 M ethylene glycol, and 0.1 M NaOH, was circulated through a circulation path C3 including the desalination chamber 30. Under these conditions, a voltage of 35 V was applied between the anode 11 and the cathode 13.

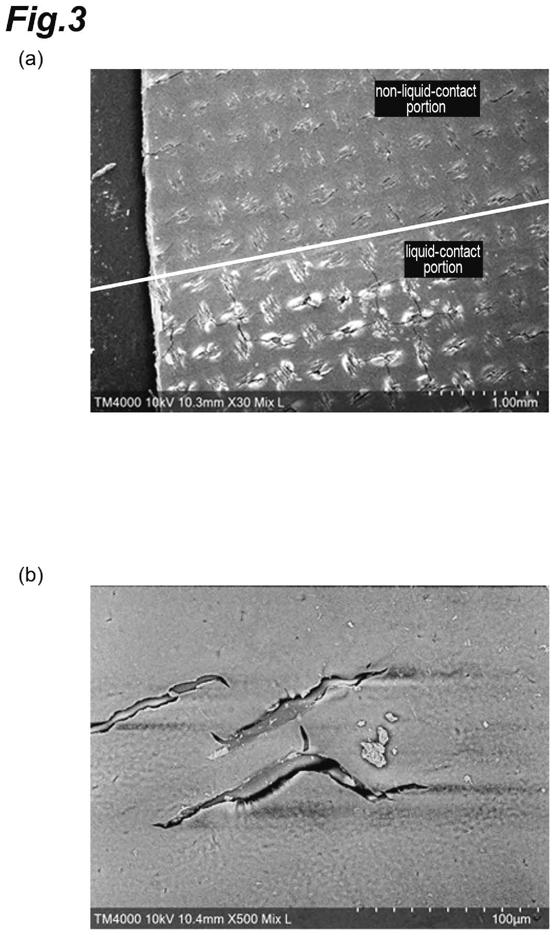

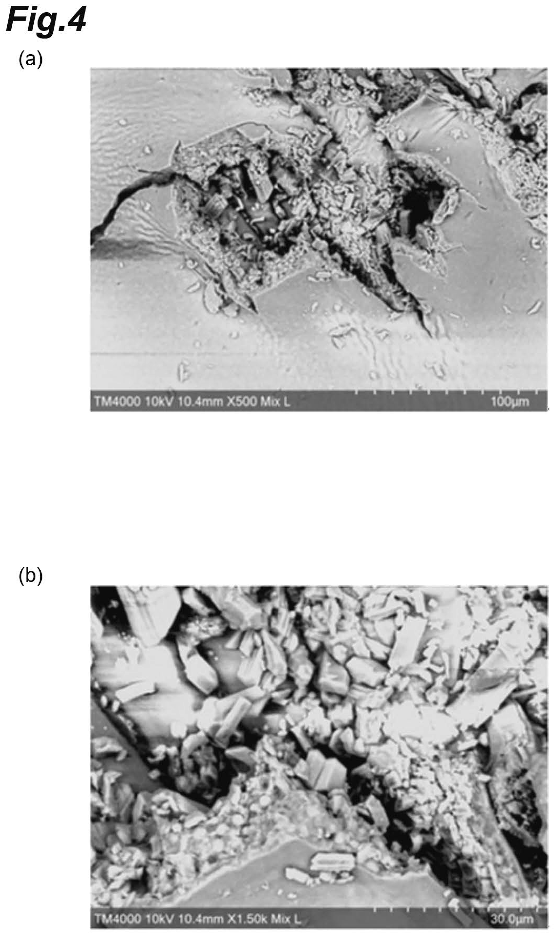

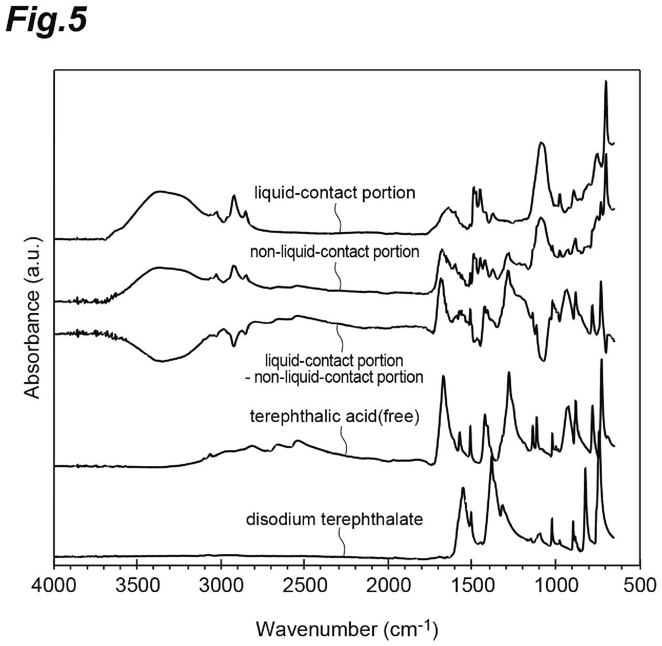

Immediately after application of the voltage, the current value decreased to about 0.1 A, and electrodialysis substantially did not proceed. The anion exchange membrane 4 taken out from the recovery apparatus 101 was observed by a scanning electron microscope. FIG. 3(a) is a scanning electron microscope image (magnification during observation: 30 times) of the removed anion exchange membrane. A liquid-contact portion is a portion that was in contact with the disodium terephthalate aqueous solution, and a non-liquid-contact portion is a portion that was not in contact with the disodium terephthalate aqueous solution. FIG. 3(b) is an enlarged view (magnification during observation: 500 times) of the non-liquid-contact portion of the anion exchange membrane. FIG. 4 shows scanning electron microscope images of the liquid-contact portion of the anion exchange membrane, in which (a) is an enlarged view at 500 times and (b) is an enlarged view at 1500 times. In the liquid-contact portion of the anion exchange membrane, crystals containing terephthalic acid were observed to be precipitated on and within the membrane, resulting in clogging of the membrane. This was considered to be because terephthalate ions changed into the free form while passing through the anion exchange membrane 4 and precipitated within the membrane. FIG. 5 shows infrared absorption spectra of the liquid-contact portion and the non-liquid-contact portion of the anion exchange membrane, a differential infrared absorption spectrum between the liquid-contact portion and the non-liquid-contact portion, and infrared absorption spectra of free terephthalic acid and disodium terephthalate. The differential infrared absorption spectrum between the liquid-contact portion and the non-liquid-contact portion of the anion exchange membrane 4 closely matched the spectrum of free terephthalic acid. This also supported clogging of the membrane due to precipitation of free terephthalic acid. From the above, it was confirmed that it is difficult to perform electrodialysis operation according to a conventional method with the membrane configuration of Comparative Example 1.

Example 1

An electrodialysis apparatus having the configuration shown in FIG. 1 was prepared. In a recovery apparatus 100 shown in FIG. 1, ten sets of ion exchange membranes (electrodialysis units), each set consisting of a bipolar membrane 2, a first cation exchange membrane 3a, and a second cation exchange membrane 3b, were sequentially arranged from the anode 11 side toward the cathode 13 side between an anode chamber 12 provided with an anode 11 and a cathode chamber 14 provided with a cathode 13. An additional bipolar membrane 2a was arranged between the second cation exchange membrane 3b constituting the electrodialysis unit 5 positioned most on the cathode 13 side and the cathode 13. An NaOH aqueous solution having a concentration of 0.5 M (1000 mL) was circulated as an electrolyte aqueous solution through a circulation path C1 including the anode chamber 12 and the cathode chamber 14. An $H_2SO_4$ aqueous solution having a concentration of 0.01 M (500 mL) was circulated through a circulation path C2 including the acid component chamber 20. An NaOH aqueous solution having a concentration of 0.01 M (500 mL) was circulated through a circulation path C4 including the alkali component chamber 40. A disodium terephthalate ($Na_2TPA$) aqueous solution having a concentration of 0.01 M (500 mL), containing 0.01 M NaOH, was circulated through a circulation path C3 including the desalination chamber 30. Under these conditions, electrodialysis was performed by applying a voltage of 15 V between the anode 11 and the cathode 13. Even at a point of 60 minutes after initiation of energization, no membrane clogging occurred, and crystals were precipitated in the desalination chamber 30. This was considered to be because terephthalate ions could not enter the cation exchange membranes. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated.

Example 2

Electrodialysis was performed under the same conditions as in Example 1, except that sodium sulfate ($Na_2SO_4$)

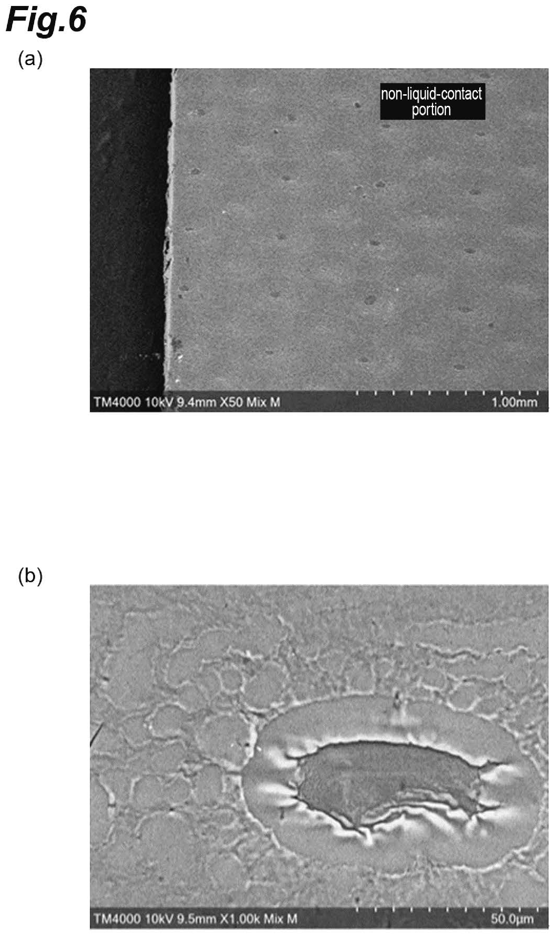

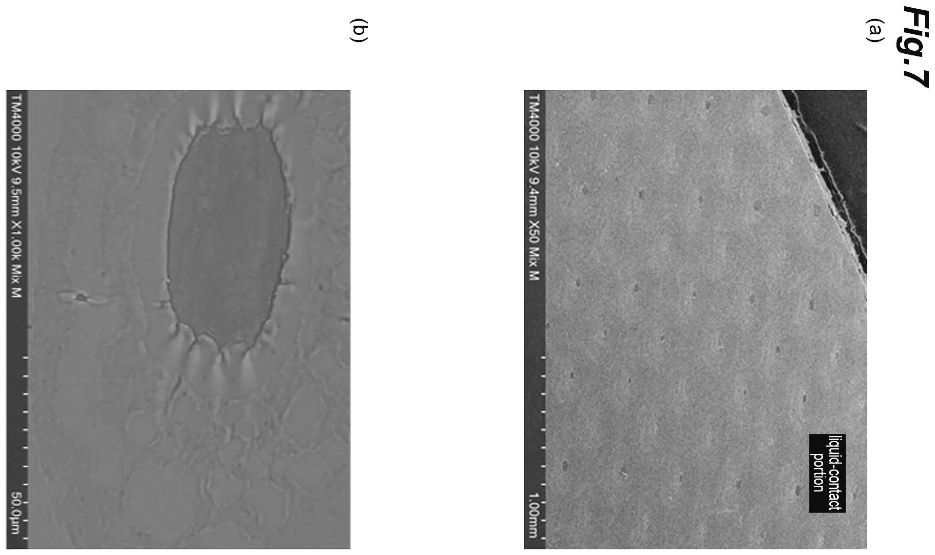

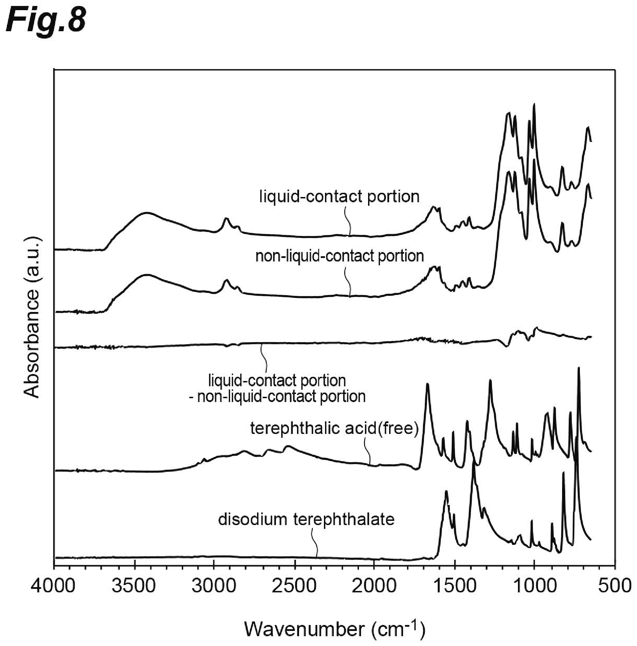

as a conductor was added to the disodium terephthalate aqueous solution (0.01 M $Na_2$TPA containing 0.01 M NaOH) circulated through the circulation path C3 including the desalination chamber 30 so as to have a concentration of 0.01 M. No membrane clogging occurred, and crystals were precipitated in the desalination chamber 30. The first cation exchange membrane 3a taken out from the recovery apparatus 100 was observed by a scanning electron microscope. FIG. 6 is a scanning electron microscope image of a non-liquid-contact portion of the first cation exchange membrane that was not in contact with the disodium terephthalate aqueous solution, in which (a) is an enlarged view at 50 times and (b) is an enlarged view at 1000 times. FIG. 7 is a scanning electron microscope image of a liquid-contact portion of the first cation exchange membrane that was in contact with the disodium terephthalate aqueous solution, in which (a) is an enlarged view at 50 times and (b) is an enlarged view at 1000 times. Even in the liquid-contact portion, no precipitation of terephthalic acid was observed within the membrane. FIG. 8 shows infrared absorption spectra of the liquid-contact portion and the non-liquid-contact portion of the first cation exchange membrane, a differential infrared absorption spectrum between the liquid-contact portion and the non-liquid-contact portion, and infrared absorption spectra of free terephthalic acid and disodium terephthalate. The differential infrared absorption spectrum between the liquid-contact portion and the non-liquid-contact portion of the first cation exchange membrane 3a did not suggest the presence of either free terephthalic acid or disodium terephthalate. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated.

Results of Examples 1 and 2

Table 1 shows conditions of electrodialysis in Examples 1 and 2.

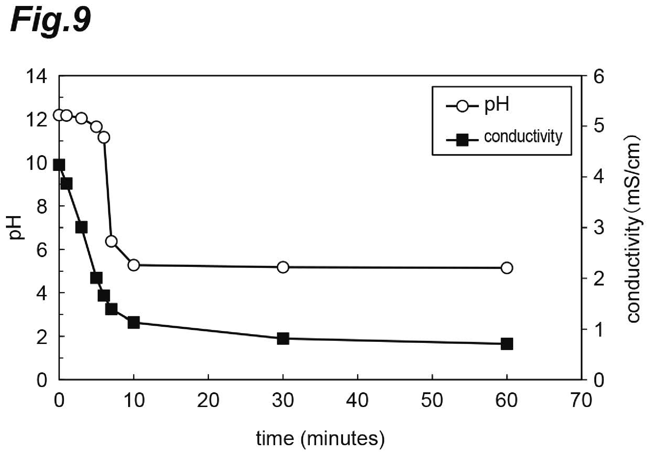

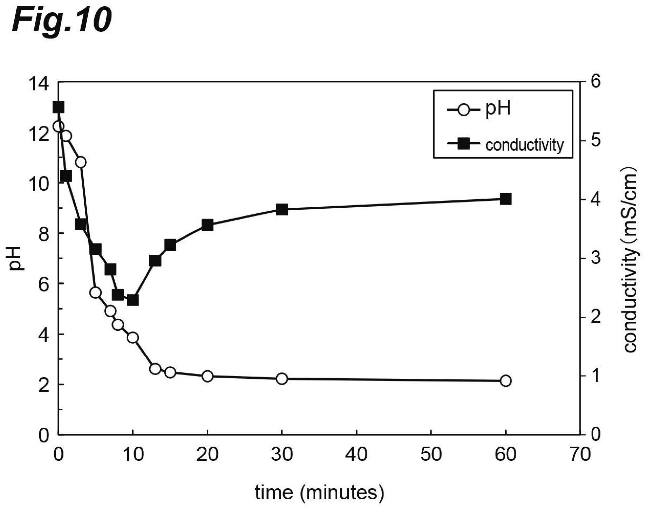

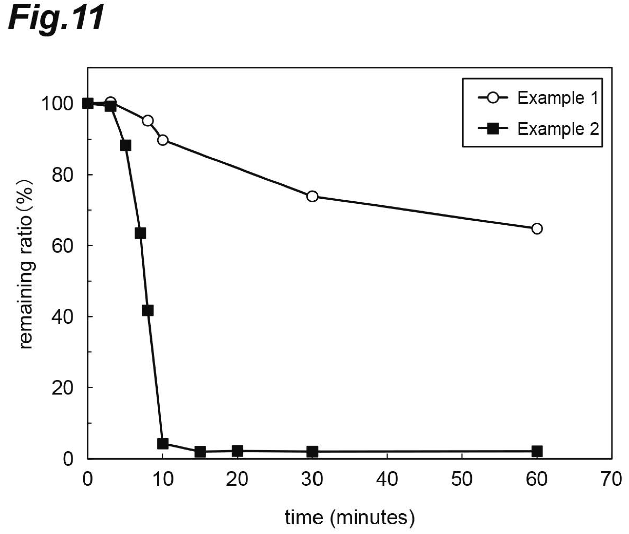

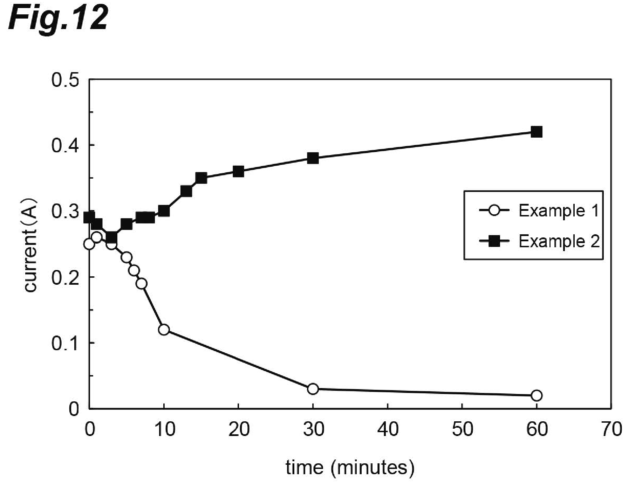

FIG. 9 is a graph showing a relationship between pH and conductivity in the desalination chamber and energization time in Example 1, and FIG. 10 is a graph showing a relationship between pH and conductivity in the desalination chamber and energization time in Example 2. FIG. 11 is a graph showing a relationship between energization time and a remaining ratio of disodium terephthalate estimated from the UV absorbance (240 nm). FIG. 12 is a graph showing a relationship between a current value flowing through the electrodialysis apparatus and energization time. In Example 1, the remaining ratio of disodium terephthalate decreased to 65% at a point of 60 minutes of energization. In Example 2, the remaining ratio of disodium terephthalate decreased to almost 0% at about 10 minutes after initiation of electrodialysis, and substantially all of the terephthalate was crystallized as free terephthalic acid in the desalination chamber. In Example 1, as shown in FIG. 9, after initiation of electrodialysis, the pH stagnated at about 5 to 6 and the conductivity decreased, and therefore the remaining ratio of disodium terephthalate was considered to have decreased relatively slowly. In Example 2, the pH rapidly decreased to 3 or less at about 10 minutes after initiation of electrodialysis, and after the conductivity decreased to 3 mS/cm, it turned to increase again. Thus, it was considered that addition of the conductor promoted a decrease in pH and maintained high conductivity for a long period of time, thereby enabling recovery of free terephthalic acid with high efficiency.

[Table 1]

| Circulation Path | | Example 1 | Example 2 |
|---|---|---|---|
| C1 | NaOH concentration [M] | 0.5 | 0.5 |
| | Solution volume [mL] | 1000 | 1000 |
| C2 | $H_2SO_4$ concentration [M] | 0.01 | 0.01 |
| | Solution volume [mL] | 500 | 500 |
| C3 | $Na_2$TPA concentration [M] | 0.01 | 0.01 |
| | NaOH concentration [M] | 0.01 | 0.01 |
| | $Na_2SO_4$ concentration [M] | - | 0.01 |
| | Solution volume [mL] | 500 | 500 |
| C4 | NaOH concentration [M] | 0.01 | 0.01 |
| | Solution volume [mL] | 500 | 500 |
| | Applied voltage [V] | 15 | 15 |

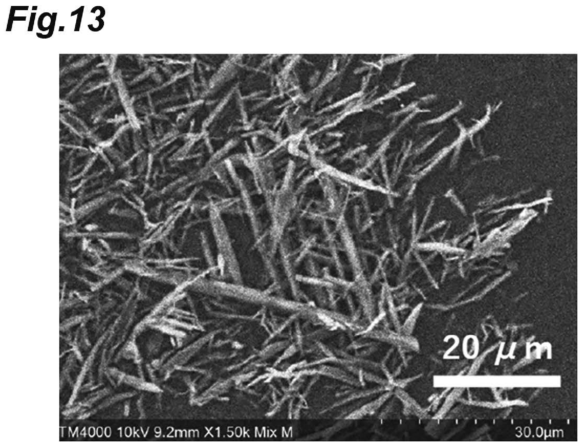

Crystals were recovered by filtration from the liquid in the desalination chamber in Example 2. It was confirmed by measurement of infrared absorption spectra that the recovered crystals were crystals of free terephthalic acid. FIG. 13 is a scanning electron microscope image of the crystals recovered in Example 2. It was revealed that large needle-shaped crystals having a major axis length (particle size) of up to 0.1 mm were obtained.

Example 3

Electrodialysis was performed under the same conditions as in Example 1, except that sodium sulfate ($Na_2SO_4$) as a conductor was added to the disodium terephthalate aqueous solution (0.01 M disodium terephthalate containing 0.01 M NaOH) circulated through the circulation path C3 including the desalination chamber 30 so as to have a concentration of 0.001 M, 0.005 M, 0.01 M, 0.02 M, 0.05 M, or 0.1 M. Under all conditions, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated.

Results of Example 3

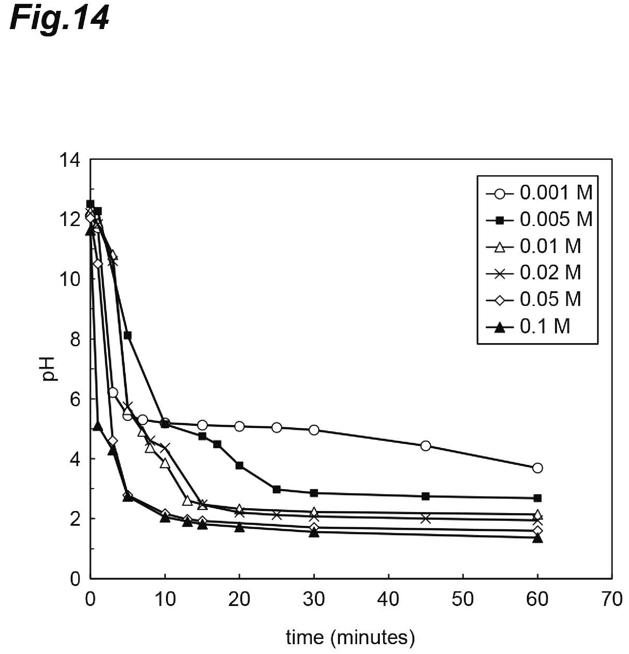

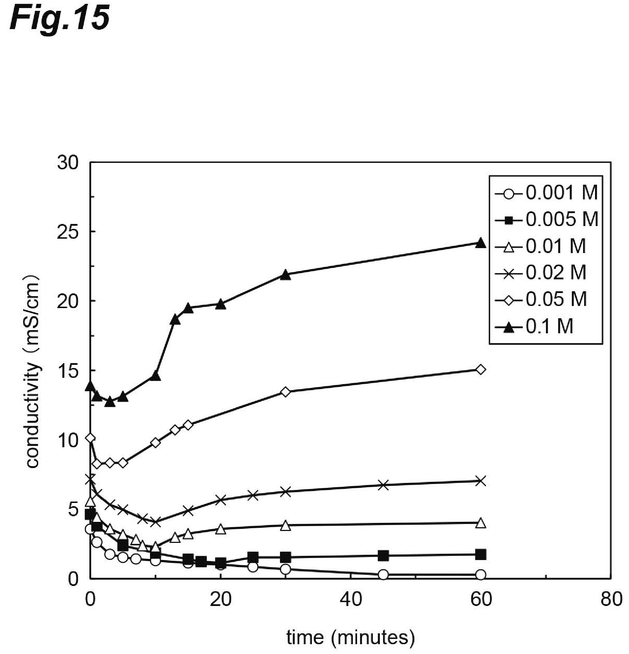

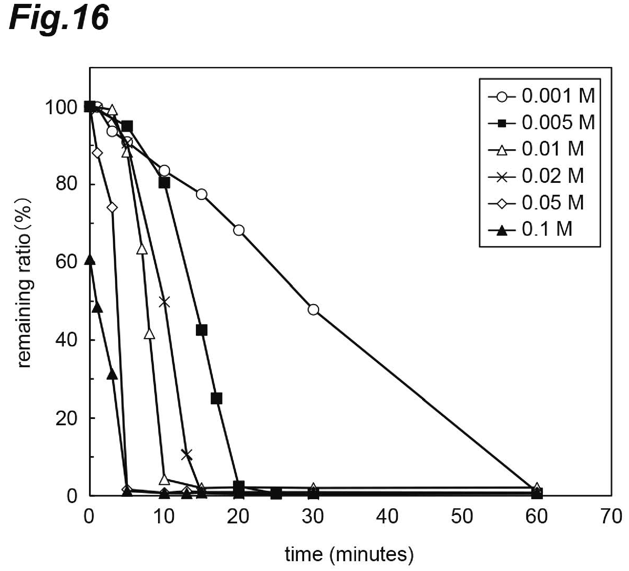

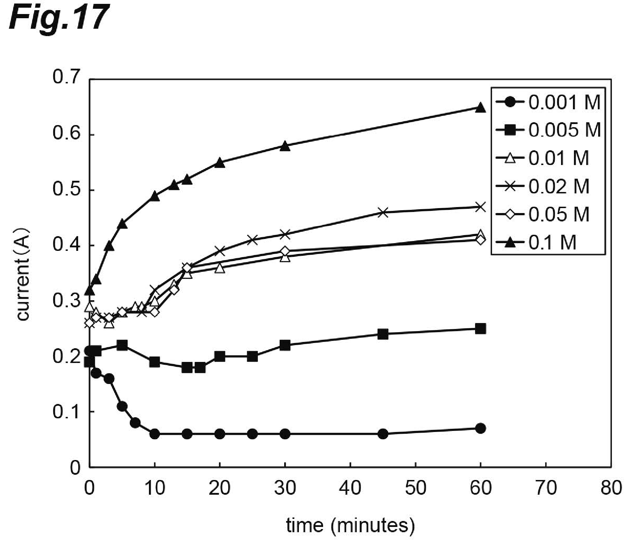

Table 2 shows conditions of electrodialysis in Example 3. FIG. 14 is a graph showing a relationship between pH in the desalination chamber and energization time. FIG. 15 is a graph showing a relationship between conductivity in the desalination chamber and energization time. FIG. 16 is a graph showing a relationship between energization time and a remaining ratio of disodium terephthalate estimated from the UV absorbance (240 nm). FIG. 17 is a graph showing a relationship between a current value flowing through the electrodialysis apparatus and energization time. From these graphs, it was suggested that as the concentration of the conductor increased, effects of promoting a decrease in pH in the desalination chamber, conductivity, and a conversion efficiency from disodium terephthalate to free terephthalic acid increased. However, even at the lowest conductor concentration of 0.001 M, conversion from disodium terephthalate to free terephthalic acid was almost 100% achieved at 60 minutes of energization.

[Table 2]

| Circulation Path | | Example 3 | | | | | |
|---|---|---|---|---|---|---|---|
| C1 | NaOH concentration [M] | 0.5 | 0.5 | 0.5 | 0.5 | 0.5 | 0.5 |
| | Solution volume [mL] | 1000 | 1000 | 1000 | 1000 | 1000 | 1000 |
| C2 | $H_2SO_4$ concentration [M] | 0.01 | 0.01 | 0.01 | 0.01 | 0.01 | 0.01 |
| | Solution volume [mL] | 500 | 500 | 500 | 500 | 500 | 500 |
| C3 | $Na_2TPA$ concentration [M] | 0.01 | 0.01 | 0.01 | 0.01 | 0.01 | 0.01 |
| | NaOH concentration [M] | 0.01 | 0.01 | 0.01 | 0.01 | 0.01 | 0.01 |
| | $Na_2SO_4$ concentration [M] | 0.001 | 0.005 | 0.01 | 0.02 | 0.05 | 0.1 |
| | Solution volume [mL] | 500 | 500 | 500 | 500 | 500 | 500 |
| C4 | NaOH concentration [M] | 0.01 | 0.01 | 0.01 | 0.01 | 0.01 | 0.01 |
| | Solution volume [mL] | 500 | 500 | 500 | 500 | 500 | 500 |
| | Applied voltage [V] | 15 | 15 | 15 | 15 | 15 | 15 |

Example 4

Electrodialysis was performed under the same conditions as in Example 1, except that sodium chloride (NaCl) as a conductor was added to the disodium terephthalate aqueous solution (0.01 M disodium terephthalate containing 0.01 M NaOH) circulated through the circulation path C3 including the desalination chamber 30 so as to have a concentration of 0.01 M, 0.05 M, or 0.1 M. No membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to

the UV absorbance (240 nm) before initiation of electrodialysis, a concentration of terephthalate ions remaining in the supernatant was calculated.

Results of Example 4

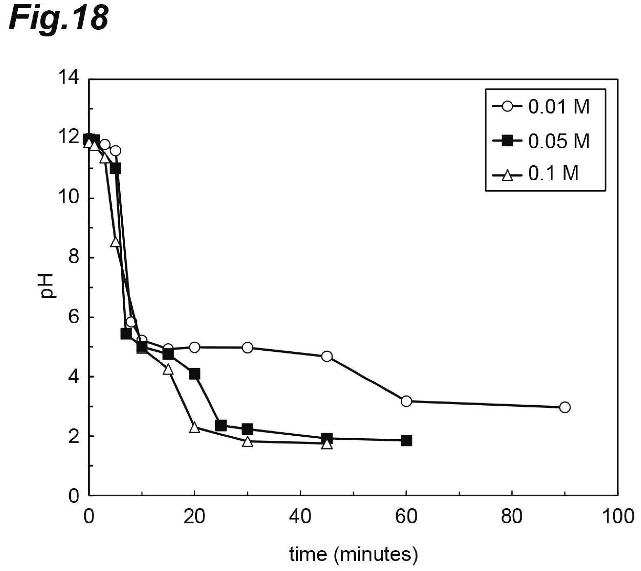

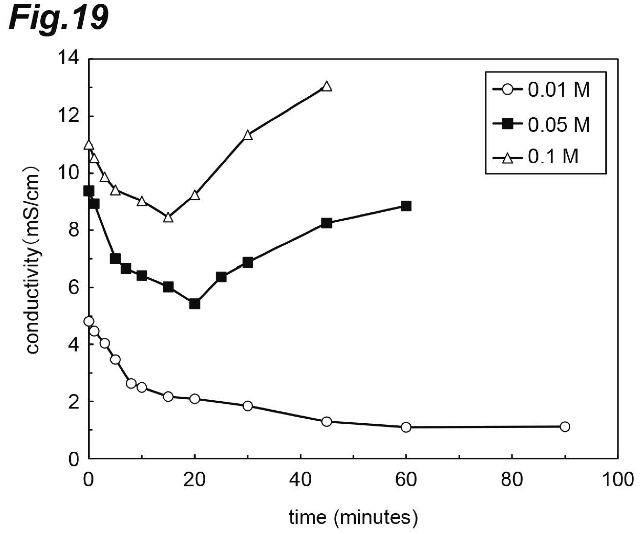

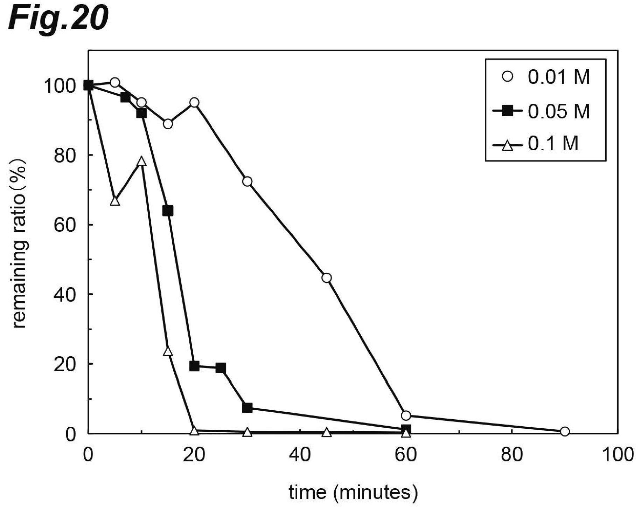

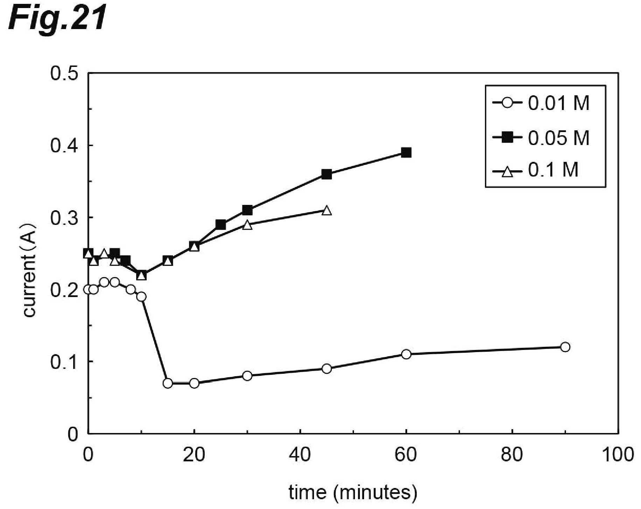

Table 3 shows conditions of electrodialysis in Example 4. FIG. 18 is a graph showing a relationship between pH in the desalination chamber and energization time in Example 4. FIG. 19 is a graph showing a relationship between conductivity in the desalination chamber and energization time. FIG. 20 is a graph showing a relationship between energization time and a remaining ratio of disodium terephthalate estimated from the UV absorbance (240 nm). FIG. 21 is a graph showing a relationship between a current value flowing through the electrodialysis apparatus and energization time. As shown in these graphs, it was confirmed that results similar to those obtained when $Na_2SO_4$ was used as the conductor were obtained even when NaCl was used as the conductor. From the results of Examples 3 and 4, it was suggested that when the pH in the desalination chamber 30 decreased to 4 or less by addition of the conductor, the conversion efficiency from disodium terephthalate to free terephthalic acid improved to nearly 100%.

[Table 3]

| Circulation Path | | Example 4 | | |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 0.01 | 0.01 | 0.01 |
| | Solution volume [mL] | 500 | 500 | 500 |
| C3 | $Na_2TPA$ concentration [M] | 0.01 | 0.01 | 0.01 |
| | NaOH concentration [M] | 0.01 | 0.01 | 0.01 |
| | NaCl concentration [M] | 0.01 | 0.05 | 0.1 |
| | Solution volume [mL] | 500 | 500 | 500 |
| C4 | NaOH concentration [M] | 0.01 | 0.01 | 0.01 |
| | Solution volume [mL] | 500 | 500 | 500 |
| | Applied voltage [V] | 15 | 15 | 15 |

Example 5

Electrodialysis was performed under the same conditions as in Example 1, except that only $NaH_2PO_4$ (sodium dihydrogen phosphate) or $NaNO_3$ (sodium nitrate) as a conductor was circulated through the circulation path C3 at the concentrations shown in Table 4.

[Table 4]

| Conductor | pH |
|---|---|
| 1 mM $NaH_2PO_4$ | 3.29 |
| 5 mM $NaH_2PO_4$ | 2.75 |
| 10 mM $NaH_2PO_4$ | 2.50 |
| 50 mM $NaH_2PO_4$ | 1.89 |
| 100 mM $NaH_2PO_4$ | 1.78 |
| 1 mM $NaNO_3$ | 3.24 |
| 5 mM $NaNO_3$ | 2.54 |
| 10 mM $NaNO_3$ | 2.28 |
| 50 mM $NaNO_3$ | 1.56 |

The pH of the liquid in the desalination chamber at a point of 60 minutes of energization is shown in Table 1. As

shown in Table 1, under all conditions, the pH of the desalination chamber became 4 or less by addition of the conductor. From this, it was suggested that $NaH_2PO_4$ or $NaNO_3$ at various concentrations also functions as a conductor that improves a conversion efficiency from disodium terephthalate to free terephthalic acid.

Example 6

Electrodialysis was performed under the same conditions as in Example 1, except that a concentration of disodium terephthalate in the disodium terephthalate aqueous solution circulated through the circulation path C3 was changed to 0.03 M (containing 0.01 M NaOH), and sodium sulfate ($Na_2SO_4$) as a conductor was added to the disodium terephthalate aqueous solution so as to have a concentration of 0.02 M. Table 5 shows conditions of electrodialysis in Example 6. No membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated.

[Table 5]

| Circulation Path | | Example 6 |
|---|---|---|
| C1 | NaOH concentration [M] | 0.5 |
| | Solution volume [mL] | 1000 |
| C2 | $H_2SO_4$ concentration [M] | 0.01 |
| | Solution volume [mL] | 500 |
| C3 | $Na_2TPA$ concentration [M] | 0.03 |
| | NaOH concentration [M] | 0.01 |
| | $Na_2SO_4$ concentration [M] | 0.02 |
| | Solution volume [mL] | 500 |
| C4 | NaOH concentration [M] | 0.01 |
| | Solution volume [mL] | 500 |
| | Applied voltage [V] | 15 |

[Table 6]

| Energization Time (min) | pH | Conductivity (mS/cm) | Remaining Ratio (%) | Current (A) |
|---|---|---|---|---|
| 0 | 12.1 | 12.13 | 100.0 | 0.33 |
| 5 | 11.34 | 8.07 | 96.6 | 0.36 |
| 10 | 5.65 | 6.94 | 87.0 | 0.26 |
| 20 | 5.12 | 5.56 | 60.6 | 0.28 |
| 25 | 4.84 | 4.62 | 34.8 | 0.32 |
| 30 | 3.84 | 3.79 | 0.6 | 0.34 |
| 35 | 2.29 | 4.93 | 0.1 | 0.36 |
| 40 | 2.10 | 5.59 | 0.3 | 0.38 |
| 60 | 1.88 | 6.52 | 0.3 | 0.41 |

Measurement results are shown in Table 6. At a point of 30 minutes of energization, disodium terephthalate was almost 100% converted into free terephthalic acid and crystallized.

Example 7

Electrodialysis was performed under the same conditions as in Example 1, except that a concentration of the $H_2SO_4$ aqueous solution circulated through the circulation path C2 was changed to 0.1 M, a concentration of disodium terephthalate in the disodium terephthalate aqueous solution circulated through the circulation path C3 was changed to 0.1 M, an amount of the disodium terephthalate aqueous solution was changed to 200 mL, NaOH was added to the disodium terephthalate aqueous solution so as to have a concentration of 0.01 M, sodium sulfate ($Na_2SO_4$) as a conductor was added to the disodium terephthalate aqueous solution so as to have a concentration of 0.02 M, and a voltage applied between the anode 11 and the cathode 13 was stepwise changed as shown in Table 8. Table 7 shows conditions of electrodialysis in Example 7. No membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated.

[Table 7]

| Circulation Path | | Example 7 |
|---|---|---|
| C1 | NaOH concentration [M] | 0.5 |
| | Solution volume [mL] | 1000 |
| C2 | $H_2SO_4$ concentration [M] | 0.1 |
| | Solution volume [mL] | 500 |
| C3 | $Na_2TPA$ concentration [M] | 0.1 |
| | NaOH concentration [M] | 0.01 |
| | $Na_2SO_4$ concentration [M] | 0.02 |
| | Solution volume [mL] | 200 |
| C4 | NaOH concentration [M] | 0.01 |
| | Solution volume [mL] | 500 |
| | Applied voltage [V] | 15.1-35.1 |

[Table 8]

| Energization Time (min) | Voltage (V) | pH | Conductivity (mS/cm) | Remaining Ratio (%) | Current (A) |
|---|---|---|---|---|---|
| 0 | 15.1 | 12.08 | 16.98 | 100.0 | 0.45 |
| 5 | 15.1 | 5.67 | 13.46 | 99.0 | 0.13 |
| 10 | 15.1 | 5.56 | 11.75 | 98.5 | 0.1 |
| 15 | 15.1 | 5.57 | 11.58 | 98.5 | 0.09 |
| 15.1 | 28 | 5.57 | 11.48 | - | 0.45 |
| 20 | 28 | 5.56 | 10.11 | - | 0.23 |
| 30 | 35.1 | 5.52 | 8.43 | 77.0 | 0.32 |
| 40 | 35.1 | 5.37 | 5.81 | 47.7 | 0.62 |
| 45 | 35.1 | 4.82 | 2.63 | 4.9 | 1.25 |
| 50 | 35.1 | 2.23 | 3.18 | 0.1 | 1.38 |
| 60 | 35.1 | 2.14 | 2.89 | 0.0 | 1.46 |

Measurement results are shown in Table 8. By increasing the voltage to 35.1 V at a point of 30 minutes of energization and further continuing energization, the pH decreased, and disodium terephthalate was almost 100% converted into free terephthalic acid and crystallized.

Example 8

Electrodialysis was performed under the same conditions as in Example 1, except that a concentration of the $H_2SO_4$ aqueous solution circulated through the circulation path C2 was changed to 1 M, an amount of the $H_2SO_4$ aqueous solution was changed to 1000 mL, a concentration of disodium terephthalate in the disodium terephthalate aqueous solution circulated through the circulation path C3 was changed to 0.4 M, an amount of the disodium terephthalate aqueous solution was changed to 100 mL, NaOH was added to the disodium terephthalate aqueous solution so as to have a concentration of 0.01 M, sodium sulfate ($Na_2SO_4$) as a conductor was added so as to have a concentration of 0.02 M, and a voltage applied between the anode 11 and the cathode 13 was stepwise changed as shown in Table 10. Table 9 shows conditions of electrodialysis in Example 8. No membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated.

[Table 9]

| Circulation Path | | Example 8 |
|---|---|---|
| C1 | NaOH concentration [M] | 0.5 |
| | Solution volume [mL] | 1000 |
| C2 | $H_2SO_4$ concentration [M] | 1 |
| | Solution volume [mL] | 1000 |
| C3 | $Na_2TPA$ concentration [M] | 0.4 |
| | NaOH concentration [M] | 0.01 |
| | $Na_2SO_4$ concentration [M] | 0.02 |
| | Solution volume [mL] | 100 |
| C4 | NaOH concentration [M] | 0.01 |
| | Solution volume [mL] | 500 |
| | Applied voltage [V] | 15.1-35.1 |

[Table 10]

| Energization Time (min) | Voltage (V) | pH | Conductivity (mS/cm) | Remaining Ratio (%) | Current (A) |
|---|---|---|---|---|---|
| 0 | 15.1 | 12.35 | 40.0 | 100 | 0.49 |
| 5 | 15.1 | 5.86 | 26.2 | 99.7 | 0.23 |
| 15 | 15.1 | 5.84 | 22.1 | 90.5 | 0.24 |
| 15.1 | 28 | 5.84 | 21.7 | - | 0.36 |
| 30 | 28 | 5.84 | 21.5 | 85.2 | 0.22 |
| 30.1 | 35.1 | 5.84 | 21.3 | - | 0.3 |
| 90 | 35.1 | 5.79 | 21.2 | 69.2 | 0.24 |
| 100 | 35.1 | 5.77 | 19.6 | - | 0.26 |
| 120 | 35.1 | 5.75 | 17.5 | - | 0.25 |
| 180 | 35.1 | 5.68 | 17.9 | 51.4 | 0.29 |
| 250 | 35.1 | 5.42 | 9.07 | 18.4 | 0.67 |
| 275 | 35.1 | 2.06 | 4.42 | 0.1 | 1.54 |

Measurement results are shown in Table 10. By increasing the voltage to 35.1 V at a point of 30 minutes of energization and further continuing energization, the pH decreased, and disodium terephthalate was almost 100%

converted into terephthalic acid and crystallized.

Example 9

Electrodialysis was performed under the same conditions as in Example 1, except that a concentration of disodium terephthalate in the disodium terephthalate aqueous solution circulated through the circulation path C3 was changed to 0.1 M, an amount of the disodium terephthalate aqueous solution was changed to 200 mL, NaOH was added to the disodium terephthalate aqueous solution so as to have a concentration of 0.025 M, sodium sulfate ($Na_2SO_4$) as a conductor was added so as to have a concentration of 0.05 M, and a voltage applied between the anode 11 and the cathode 13 was set to 35 V.

Example 10

Electrodialysis was performed under the same conditions as in Example 9, except that a concentration of the $H_2SO_4$ aqueous solution circulated through the circulation path C2 was changed to 0.1 M.

Example 11

Electrodialysis was performed under the same conditions as in Example 9, except that a concentration of $Na_2SO_4$ (conductor) in the disodium terephthalate aqueous solution circulated through the circulation path C3 was changed to 0.1 M.

Results of Examples 9 to 11

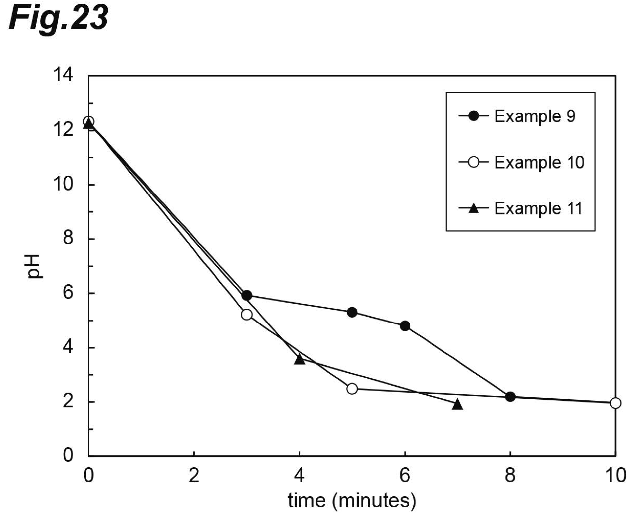

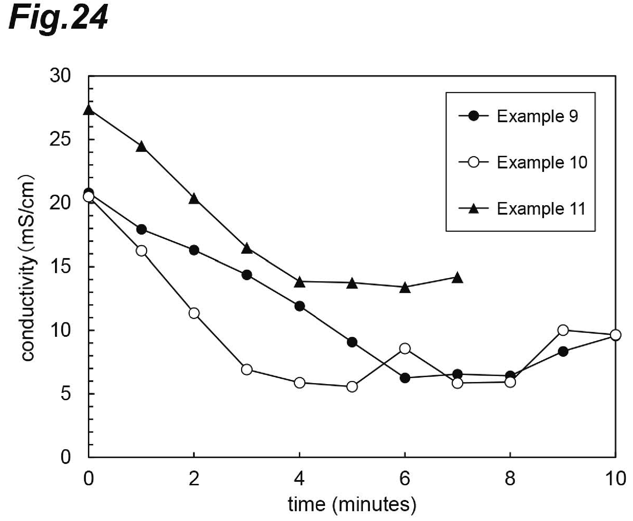

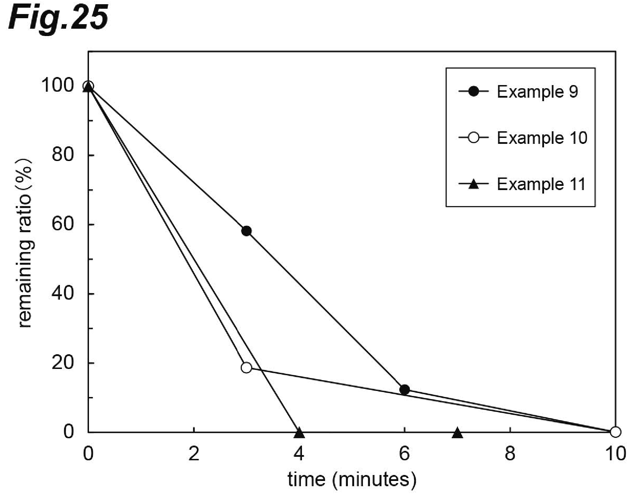

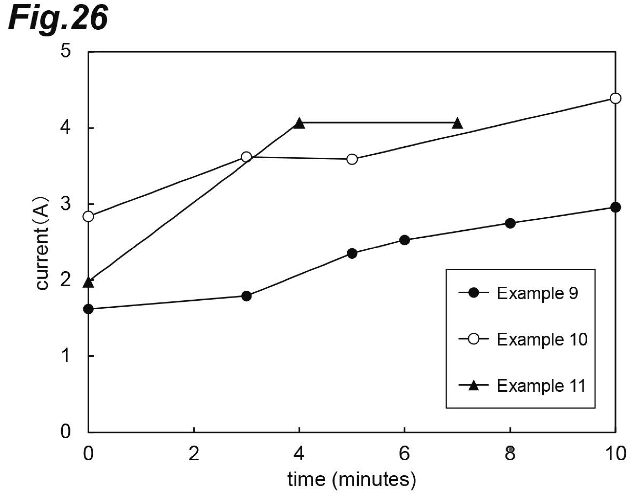

Table 11 shows conditions of electrodialysis tests in Examples 9 to 11. In the electrodialysis tests of Examples 9 to 11, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated. FIG. 23 is a graph showing a relationship between pH in the desalination chamber and energization time. FIG. 24 is a graph showing a relationship between conductivity in the desalination chamber and energization time. FIG. 25 is a graph showing a relationship between energization time and a remaining ratio of disodium terephthalate. FIG. 26 is a graph showing a relationship between a current value flowing through the electrodialysis apparatus and energization time. As shown in FIG. 25, under all conditions, 100% conversion from disodium terephthalate to free terephthalic acid was achieved in a short period of time. From comparison between Example 9 and Example 10, it was confirmed that as a concentration of the $H_2SO_4$ aqueous solution circulated through the circulation path C2 increased, a conversion efficiency from disodium terephthalate to free terephthalic acid tended to increase. From comparison among Examples 9 to 11, it was confirmed that as a concentration of the conductor in the desalination chamber 30 increased, a decrease in pH in the desalination chamber tended to be promoted. When the conversion efficiency from disodium terephthalate to free terephthalic acid is high, crystallization of terephthalic acid can be completed in a shorter time.

[Table 11]

| Circulation Path | | Example 9 | Example 10 | Example 11 |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 0.01 | 0.1 | 0.1 |
| | Solution volume [mL] | 500 | 500 | 500 |
| C3 | $Na_2TPA$ concentration [M] | 0.1 | 0.1 | 0.1 |
| | NaOH concentration [M] | 0.025 | 0.025 | 0.025 |
| | $Na_2SO_4$ concentration [M] | 0.05 | 0.05 | 0.1 |
| | Solution volume [mL] | 200 | 200 | 200 |

(continued)

| Circulation Path | | Example 9 | Example 10 | Example 11 |
|---|---|---|---|---|
| C4 | NaOH concentration [M] | 0.01 | 0.01 | 0.01 |
| | Solution volume [mL] | 500 | 500 | 500 |
| | Applied voltage [V] | 35 | 35 | 35 |

Example 12

Electrodialysis was performed under the same conditions as in Example 1, except that a concentration of the $H_2SO_4$ aqueous solution circulated through the circulation path C2 was changed to 1 M, a concentration of disodium terephthalate in the disodium terephthalate aqueous solution circulated through the circulation path C3 was changed to 0.4 M, an amount of the disodium terephthalate aqueous solution was changed to 100 mL, NaOH was added to the disodium terephthalate aqueous solution so as to have a concentration of 0.1 M, sodium sulfate ($Na_2SO_4$) was added to the disodium terephthalate aqueous solution so as to have a concentration of 0.02 M, and a voltage applied between the anode 11 and the cathode 13 was set to 35 V.

Example 13

Electrodialysis was performed under the same conditions as in Example 12, except that a concentration of $Na_2SO_4$ in the disodium terephthalate aqueous solution circulated through the circulation path C3 was changed to 0.05 M.

Example 14

Electrodialysis was performed under the same conditions as in Example 12, except that a concentration of $Na_2SO_4$ in the disodium terephthalate aqueous solution circulated through the circulation path C3 was changed to 0.1 M.

Results of Examples 12 to 14

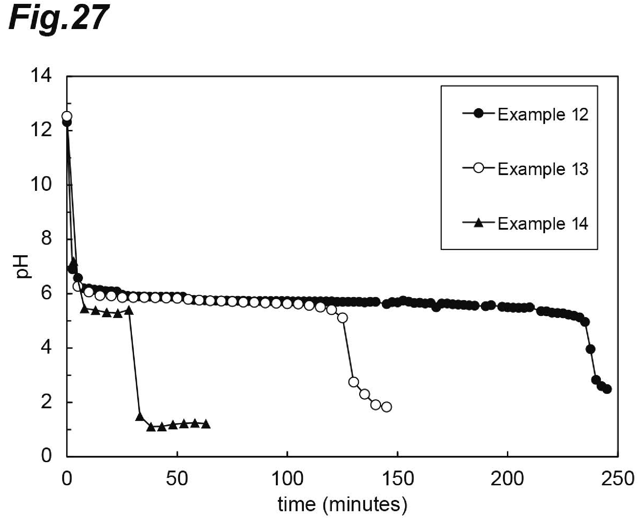

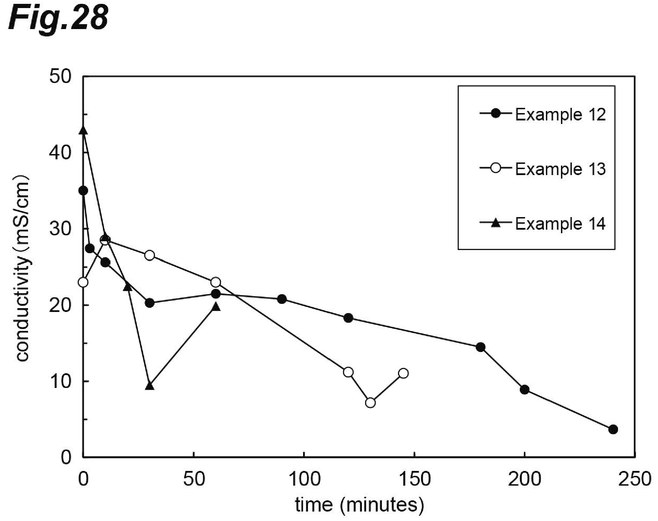

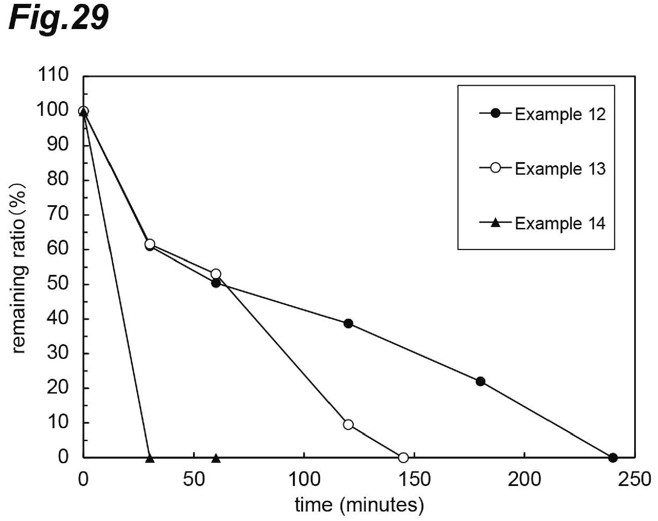

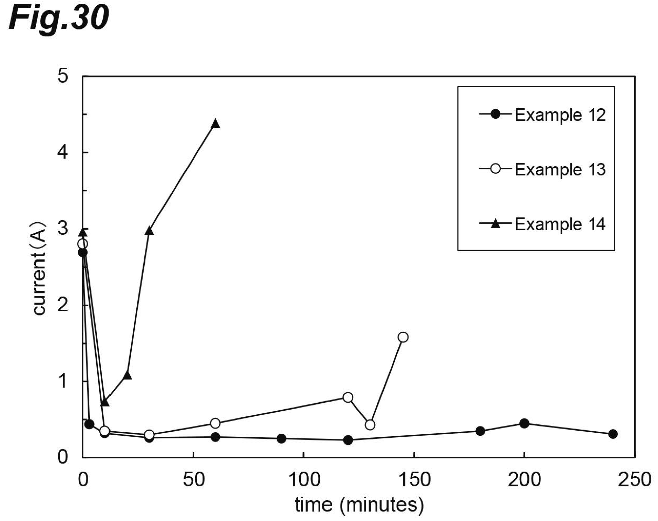

Table 12 shows conditions of electrodialysis tests in Examples 12 to 14. In the electrodialysis tests of Examples 12 to 14, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated. FIG. 27 is a graph showing a relationship between pH in the desalination chamber and energization time. FIG. 28 is a graph showing a relationship between conductivity in the desalination chamber and energization time. FIG. 29 is a graph showing a relationship between energization time and a remaining ratio of disodium terephthalate. FIG. 30 is a graph showing a relationship between a current value flowing through the electrodialysis apparatus and energization time. From these graphs, it was confirmed that as the concentration of the conductor increased, a decrease in pH in the desalination chamber was promoted, and a conversion efficiency from disodium terephthalate to free terephthalic acid increased. That is, it was suggested that crystallization of terephthalic acid can be completed in a shorter time.

[Table 12]

| Circulation Path | | Example 12 | Example 13 | Example 14 |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 1 | 1 | 1 |
| | Solution volume [mL] | 500 | 500 | 500 |
| C3 | $Na_2TPA$ concentration [M] | 0.4 | 0.4 | 0.4 |
| | NaOH concentration [M] | 0.1 | 0.1 | 0.1 |
| | $Na_2SO_4$ concentration [M] | 0.02 | 0.05 | 0.1 |
| | Solution volume [mL] | 100 | 100 | 100 |

(continued)

| Circulation Path | | Example 12 | Example 13 | Example 14 |
|---|---|---|---|---|
| C4 | NaOH concentration [M] | 0.01 | 0.01 | 0.01 |
| | Solution volume [mL] | 500 | 500 | 500 |
| | Applied voltage [V] | 35 | 35 | 35 |

Example 15

Electrodialysis was performed under the same conditions as in Example 1, except that a concentration of the $H_2SO_4$ aqueous solution circulated through the circulation path C2 was changed to 0.1 M, a concentration of disodium terephthalate in the disodium terephthalate aqueous solution circulated through the circulation path C3 was changed to 0.1 M, an amount of the disodium terephthalate aqueous solution was changed to 200 mL, NaOH was added to the disodium terephthalate aqueous solution so as to have a concentration of 0.1 M, and sodium sulfate ($Na_2SO_4$) was added to the disodium terephthalate aqueous solution so as to have a concentration of 0.02 M.

Example 16

Electrodialysis was performed under the same conditions as in Example 15, except that a concentration of $Na_2SO_4$ in the disodium terephthalate aqueous solution circulated through the circulation path C3 was changed to 0.05 M.

Example 17

Electrodialysis was performed under the same conditions as in Example 15, except that a concentration of $Na_2SO_4$ in the disodium terephthalate aqueous solution circulated through the circulation path C3 was changed to 0.1 M.

Results of Examples 15 to 17

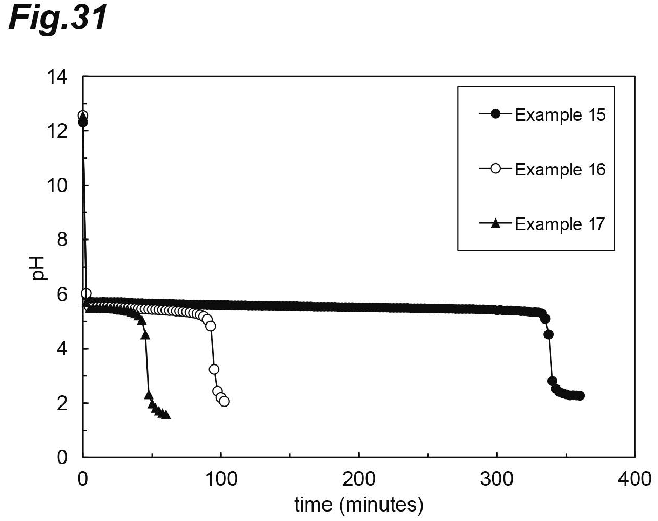

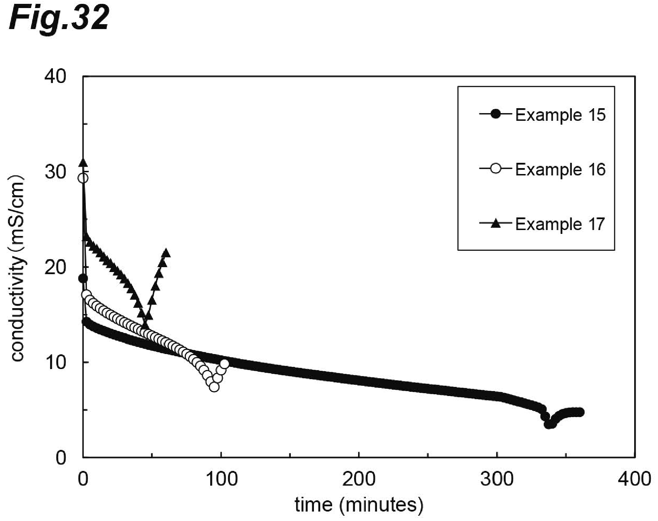

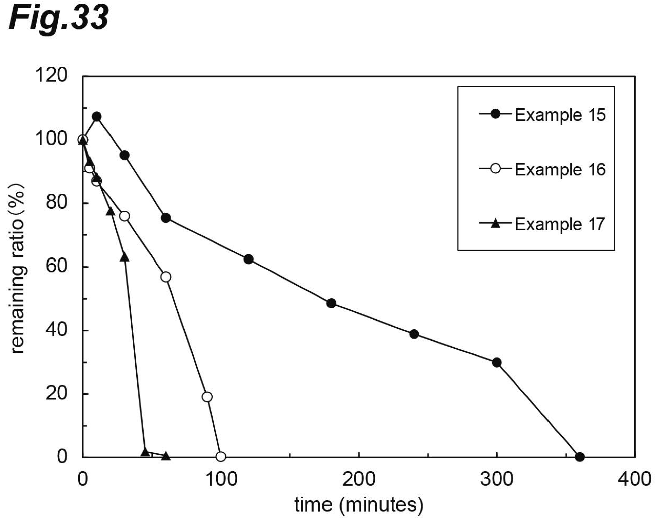

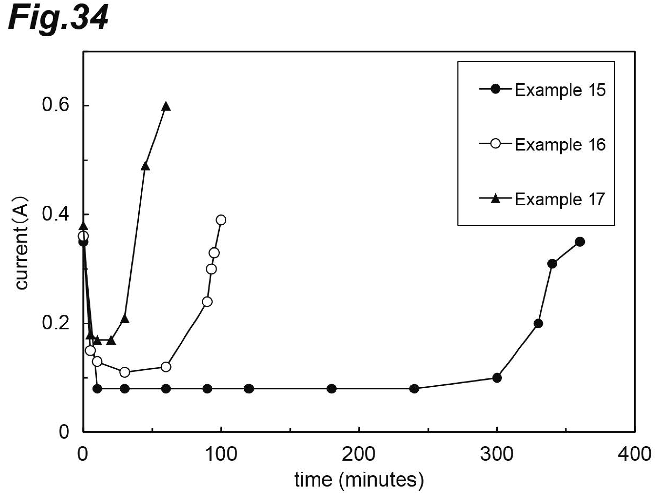

Table 13 shows conditions of electrodialysis tests in Examples 15 to 17. In the electrodialysis tests of Examples 15 to 17, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated. FIG. 31 is a graph showing a relationship between pH in the desalination chamber and energization time. FIG. 32 is a graph showing a relationship between conductivity in the desalination chamber and energization time. FIG. 33 is a graph showing a relationship between energization time and a remaining ratio of disodium terephthalate. FIG. 34 is a graph showing a relationship between a current value flowing through the electrodialysis apparatus and energization time. From these graphs, it was confirmed that as the concentration of the conductor increased, a decrease in pH in the desalination chamber was promoted, and a conversion efficiency from disodium terephthalate to free terephthalic acid increased. That is, it was suggested that crystallization of terephthalic acid can be completed in a shorter time.

[Table 13]

| Circulation Path | | Example 15 | Example 16 | Example 17 |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 0.1 | 0.1 | 0.1 |
| | Solution volume [mL] | 500 | 500 | 500 |
| C3 | $Na_2TPA$ concentration [M] | 0.1 | 0.1 | 0.1 |
| | NaOH concentration [M] | 0.1 | 0.1 | 0.1 |
| | $Na_2SO_4$ concentration [M] | 0.02 | 0.05 | 0.1 |
| | Solution volume [mL] | 200 | 200 | 200 |

(continued)

| Circulation Path | | Example 15 | Example 16 | Example 17 |
|---|---|---|---|---|
| C4 | NaOH concentration [M] | 0.01 | 0.01 | 0.01 |
| | Solution volume [mL] | 500 | 500 | 500 |
| | Applied voltage [V] | 15 | 15 | 15 |

Example 18

Electrodialysis was performed under the same conditions as in Example 16, except that a concentration of the $H_2SO_4$ aqueous solution circulated through the circulation path C2 was changed to 0.01 M.

Example 19

Electrodialysis was performed under the same conditions as in Example 16, except that a concentration of the $H_2SO_4$ aqueous solution circulated through the circulation path C2 was changed to 1 M.

Results of Examples 16, 18, and 19

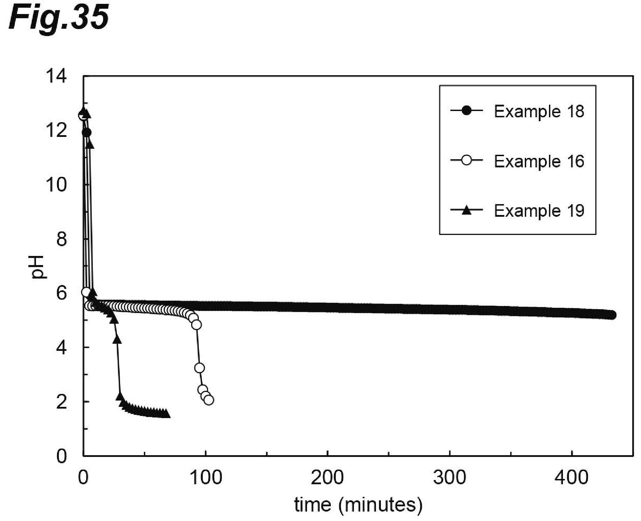

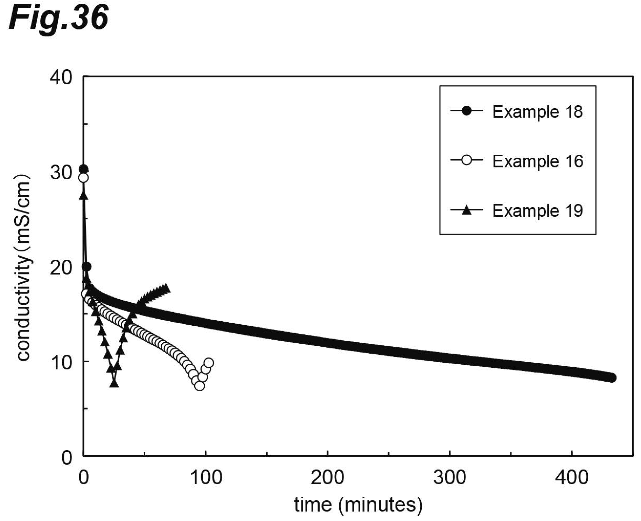

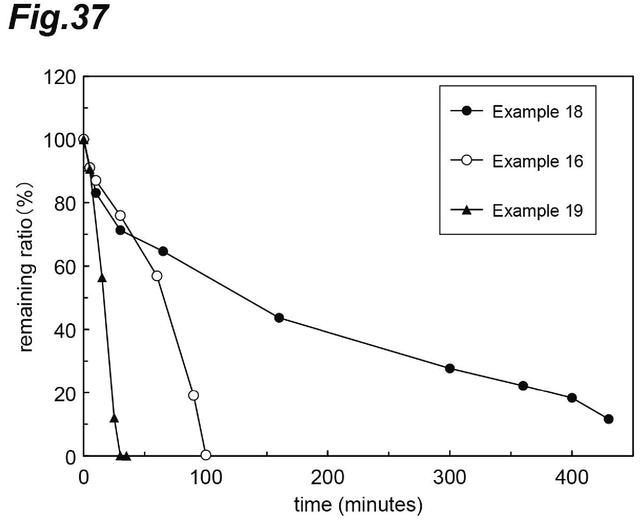

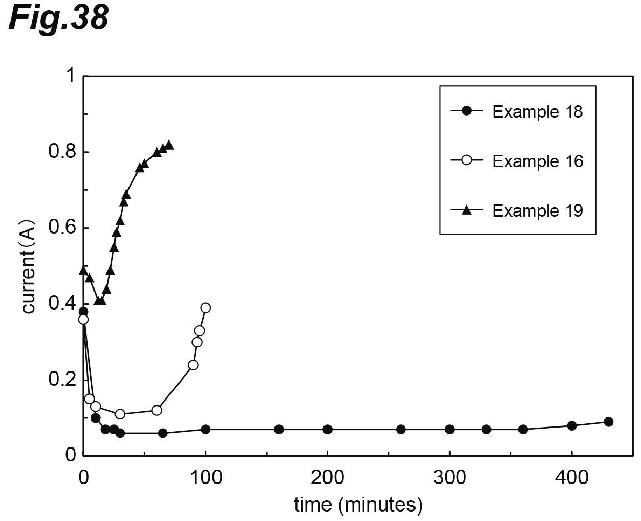

Table 14 shows conditions of electrodialysis tests in Examples 16, 18, and 19. Results of Example 16 are as described in the previous section "Results of Examples 15 to 17." In the electrodialysis tests of Examples 18 and 19, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated. FIG. 35 is a graph showing a relationship between pH in the desalination chamber and energization time. FIG. 36 is a graph showing a relationship between conductivity in the desalination chamber and energization time. FIG. 37 is a graph showing a relationship between energization time and a remaining ratio of disodium terephthalate. FIG. 38 is a graph showing a relationship between a current value flowing through the electrodialysis apparatus and energization time. From these graphs, it was confirmed that as the concentration of the $H_2SO_4$ aqueous solution in the acid component chamber increased, a decrease in pH in the desalination chamber was promoted, and a conversion efficiency from disodium terephthalate to free terephthalic acid increased. That is, it was suggested that crystallization of terephthalic acid can be completed in a shorter time.

[Table 14]

| Circulation Path | | Example 16 | Example 18 | Example 19 |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 0.1 | 0.01 | 1 |
| | Solution volume [mL] | 500 | 500 | 500 |
| C3 | $Na_2TPA$ concentration [M] | 0.1 | 0.1 | 0.1 |
| | NaOH concentration [M] | 0.1 | 0.1 | 0.1 |
| | $Na_2SO_4$ concentration [M] | 0.05 | 0.05 | 0.05 |
| | Solution volume [mL] | 200 | 200 | 200 |
| C4 | NaOH concentration [M] | 0.01 | 0.01 | 0.01 |
| | Solution volume [mL] | 500 | 500 | 500 |
| | Applied voltage [V] | 15 | 15 | 15 |

Example 20

Electrodialysis was performed under the same conditions as in Example 16, except that a voltage applied between the anode 11 and the cathode 13 was set to 20 V.

Example 21

Electrodialysis was performed under the same conditions as in Example 16, except that a voltage applied between the anode 11 and the cathode 13 was set to 28 V.

Results of Examples 16, 20, and 21

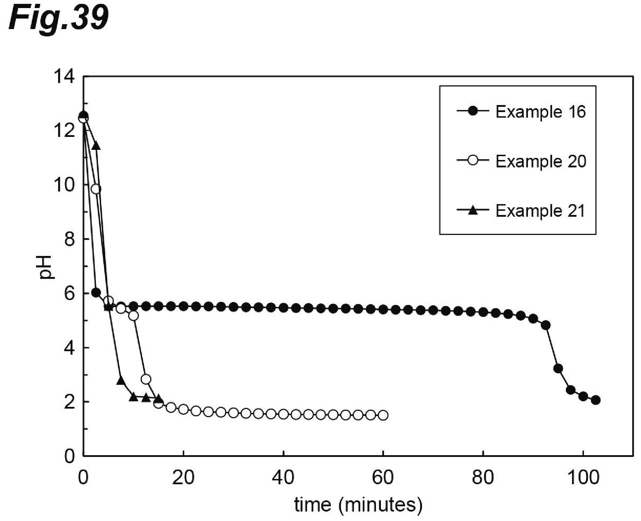

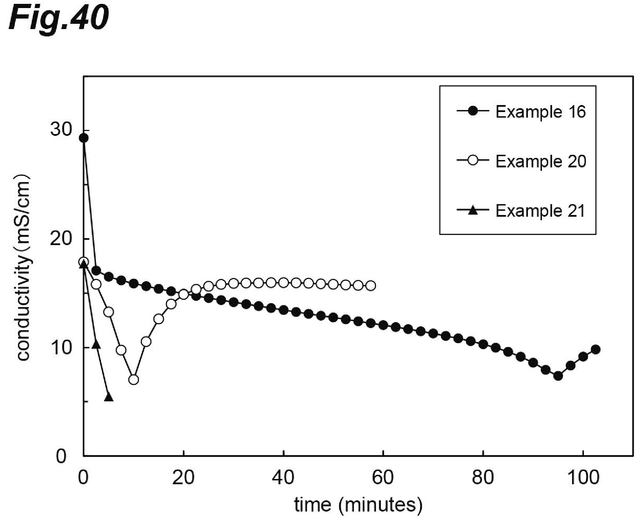

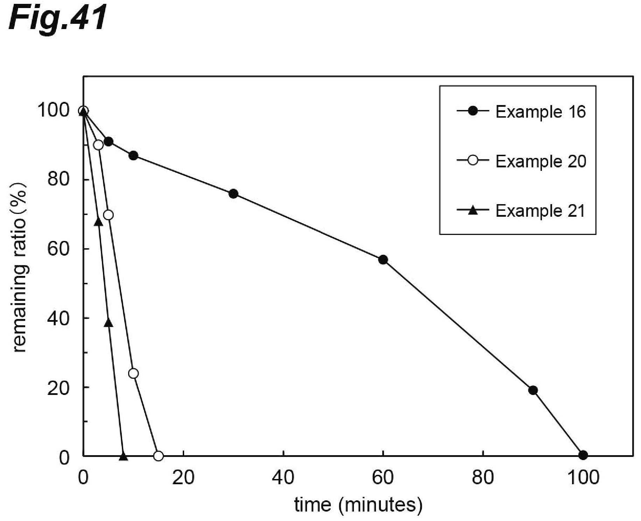

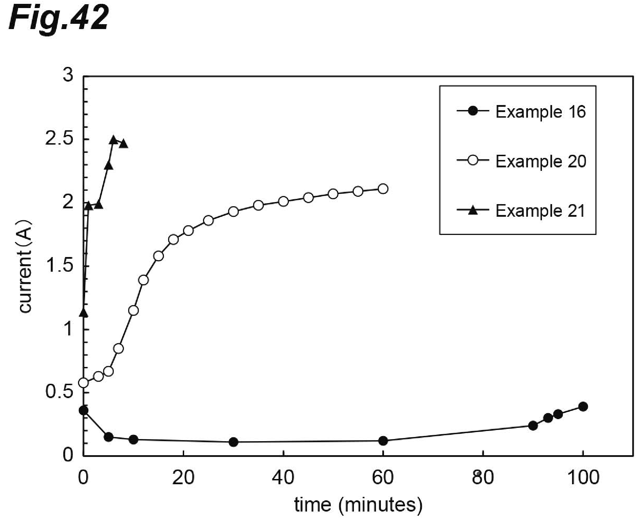

Table 15 shows conditions of electrodialysis tests in Examples 16, 20, and 21. Results of Example 16 are as described in the previous section "Results of Examples 15 to 17." In the electrodialysis tests of Examples 20 and 21, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated. FIG. 39 is a graph showing a relationship between pH in the desalination chamber and energization time. FIG. 40 is a graph showing a relationship between conductivity in the desalination chamber and energization time. FIG. 41 is a graph showing a relationship between energization time and a remaining ratio of disodium terephthalate. FIG. 42 is a graph showing a relationship between a current value flowing through the electrodialysis apparatus and energization time. From these graphs, it was confirmed that when a constant voltage is applied between the anode 11 and the cathode 13, as the applied voltage increases, a decrease in pH in the desalination chamber is promoted, and a conversion efficiency from disodium terephthalate to free terephthalic acid increases. That is, it was suggested that crystallization of terephthalic acid can be completed in a shorter time.

[Table 15]

| Circulation Path | | Example 16 | Example 20 | Example 21 |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 0.1 | 0.1 | 0.1 |
| | Solution volume [mL] | 500 | 500 | 500 |
| C3 | $Na_2TPA$ concentration [M] | 0.1 | 0.1 | 0.1 |
| | NaOH concentration [M] | 0.1 | 0.1 | 0.1 |
| | $Na_2SO_4$ concentration [M] | 0.05 | 0.05 | 0.05 |
| | Solution volume [mL] | 200 | 200 | 200 |
| C4 | NaOH concentration [M] | 0.01 | 0.01 | 0.01 |
| | Solution volume [mL] | 500 | 500 | 500 |
| | Applied voltage [V] | 15 | 20 | 28 |

In electrodialysis of Example 20, a concentration of hydroxide ions ($OH^-$) in the NaOH aqueous solution circulated through the circulation path C4 was calculated by acid-base titration at around a point of 60 minutes of energization. From a volume of the NaOH aqueous solution and the hydroxide ion concentration, an amount of hydroxide ions increased by electrodialysis was calculated to be 81.4 mmol. The increase in hydroxide ions is attributed to migration of sodium ions ($Na^+$) derived from disodium terephthalate, NaOH, and $Na_2SO_4$ contained in the disodium terephthalate aqueous solution before energization for electrodialysis into the alkali component chamber 40 by electrodialysis, together with production of NaOH in the alkali component chamber 40 by hydroxide ions ($OH^-$) supplied from the bipolar membrane. Since the disodium terephthalate aqueous solution before energization for electrodialysis contained 80 mmol of sodium ions ($Na^+$) derived from disodium terephthalate, NaOH, and $Na_2SO_4$, it was confirmed that almost 100% of sodium ions ($Na^+$) in the disodium terephthalate aqueous solution were transferred into the alkali component chamber 40 and converted into NaOH

by electrodialysis. That is, it was demonstrated that by electrodialysis, crystals of free terephthalic acid can be produced and recovered in the desalination chamber 30, and at the same time, NaOH can be produced and recovered in the alkali component chamber 40 from sodium ions ($Na^+$) contained in the desalination chamber.

Example 22

Depolymerization of Polyethylene Terephthalate (PET) Recovered waste PET bottles were washed, flaked, and freeze-ground to prepare a granular PET sample. Methanol (125 g) was placed in a tabletop kneader (PNV-1, manufactured by Irie Shokai Co., Ltd.). Sodium hydroxide (114 g, 2.85 mol) and the PET sample (250 g; amount in moles based on a repeating unit: 1.30 mol) were added to methanol at room temperature (27°C) in the kneader, thereby forming a powdery mixture containing the PET sample, sodium hydroxide, and methanol. The resulting powdery mixture was kneaded at a rotational speed of 50 rpm for 9 minutes without heating. After kneading was stopped, disodium terephthalate and ethylene glycol generated by the depolymerization reaction were dissolved in 2.3 L of water. The aqueous solution containing solids was filtered to recover solids (unreacted PET sample) and a filtrate (depolymerization liquid).

Depolymerization Ratio

The solids recovered from the aqueous solution were dried by heating in an oven at 100°C for 12 hours or more. The mass W_fin (g) of the dried solids (unreacted PET sample) was measured. A depolymerization ratio was calculated using the following equation. The depolymerization ratio was 99.4%.

$$\text{Depolymerization ratio (\%)} = [(W_int - W_fin) / W_int] \times 100$$

In the above equation, W_int represents a mass (g) of the PET sample subjected to depolymerization.

Terephthalic Acid Conversion Ratio

A portion of the depolymerization liquid, which was a filtrate obtained by removing solids from the aqueous solution after depolymerization, was diluted 250 times with a diluent (1 N HCl aqueous solution : isopropanol = 1:9 (v/v)) to obtain an analytical sample solution. The obtained sample solution was analyzed by HPLC under the following conditions to quantify terephthalic acid in the depolymerization liquid. Column: L-column 2 ODS, 2.1 mm × 150 mm, particle size 2 μm (Chemicals Evaluation and Research Institute)

Column temperature: 35°C
Mobile phase A: water containing 0.1% acetic acid
Mobile phase B: acetonitrile containing 0.1% acetic acid
Flow rate: 0.2 mL/min
Linear gradient: 15% B (0 min) → 80% B (26 min)
Detector: photodiode array (wavelength 190-600 nm)

Based on quantification results by HPLC, a terephthalic acid conversion ratio was calculated using the following equation.

Terephthalic acid conversion ratio (%) = [(W_tpa / 166) / (W_int / 192)] × 100

In the above equation, W_int represents the mass (g) of the PET sample subjected to depolymerization, and W_tpa represents a mass (g), in terms of a free terephthalic acid equivalent, of terephthalic acid generated by depolymerization calculated based on the HPLC quantification results. The value 166 represents the molecular weight of terephthalic acid, and the value 192 represents a formula weight of a repeating unit ($C_{10}H_8O_4$) of PET. The terephthalic acid conversion ratio was 101%. The concentration of disodium terephthalate in the depolymerization liquid was 0.54 M. The solution contained 0.54 M ethylene glycol (EG) generated by depolymerization, 0.1 M excess NaOH not consumed in the depolymerization reaction, and 1.62 M methanol (MeOH).

Electrodialysis Test

The depolymerization liquid obtained by removing solids from the aqueous solution after depolymerization was diluted with water so that the concentration of disodium terephthalate became 0.1 M, thereby obtaining a depolymerization

liquid for electrodialysis. Sodium sulfate ($Na_2SO_4$) was added to the depolymerization liquid for electrodialysis so as to have a concentration of 0.05 M. An electrodialysis test was performed under the same conditions as in Example 1, except that 200 mL of the depolymerization liquid for electrodialysis was circulated through the circulation path C3 and the concentration of the $H_2SO_4$ aqueous solution circulated through the circulation path C2 was changed to 0.1 M.

Example 23

The depolymerization liquid obtained in Example 22 was diluted with water so that the concentration of disodium terephthalate became 0.4 M, thereby obtaining a depolymerization liquid for electrodialysis. Sodium sulfate ($Na_2SO_4$) was added to the depolymerization liquid for electrodialysis so as to have a concentration of 0.05 M. An electrodialysis test was performed under the same conditions as in Example 1, except that 100 mL of the depolymerization liquid for electrodialysis was circulated through the circulation path C3, the concentration of the $H_2SO_4$ aqueous solution circulated through the circulation path C2 was changed to 1 M, and a voltage applied between the anode 11 and the cathode 13 was set to 35 V.

Example 24

Sodium sulfate ($Na_2SO_4$) was added to the depolymerization liquid obtained in Example 22 so as to have a concentration of 0.2 M, thereby preparing a depolymerization liquid for electrodialysis. An electrodialysis test was performed under the same conditions as in Example 1, except that 100 mL of the depolymerization liquid for electrodialysis was circulated through the circulation path C3, the concentration of the $H_2SO_4$ aqueous solution circulated through the circulation path C2 was changed to 1 M, and a voltage applied between the anode 11 and the cathode 13 was set to 35 V.

Results of Examples 22, 23, and 24

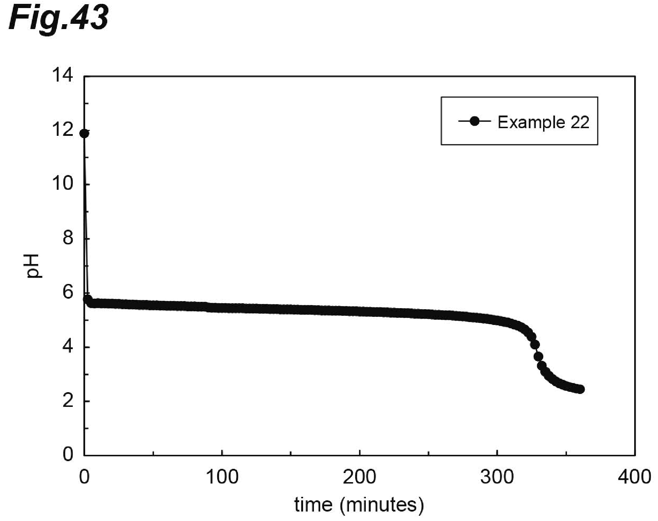

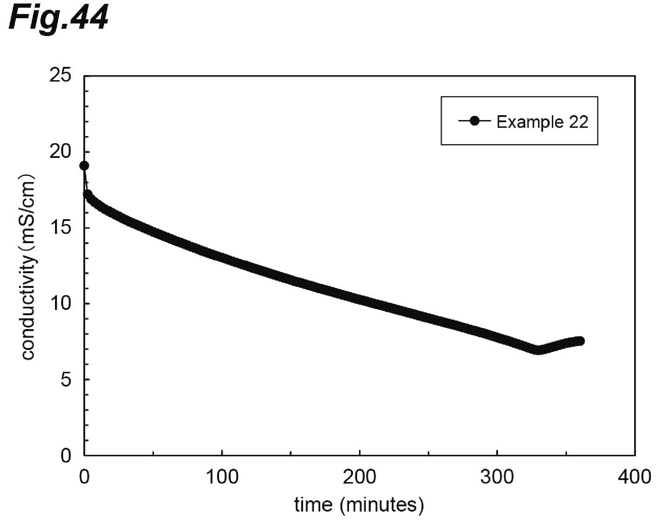

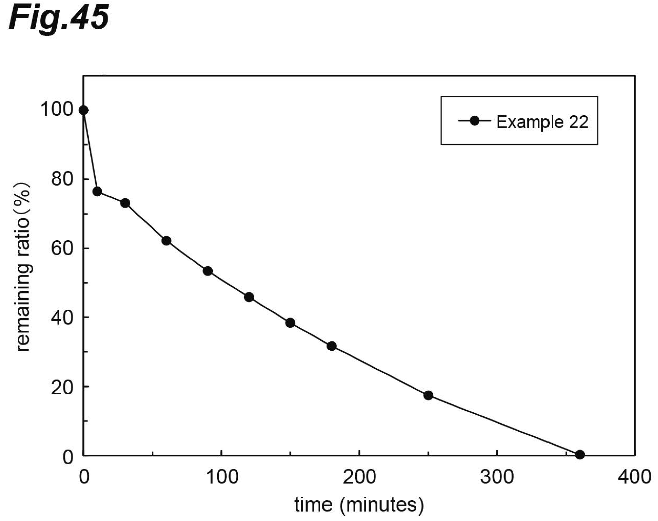

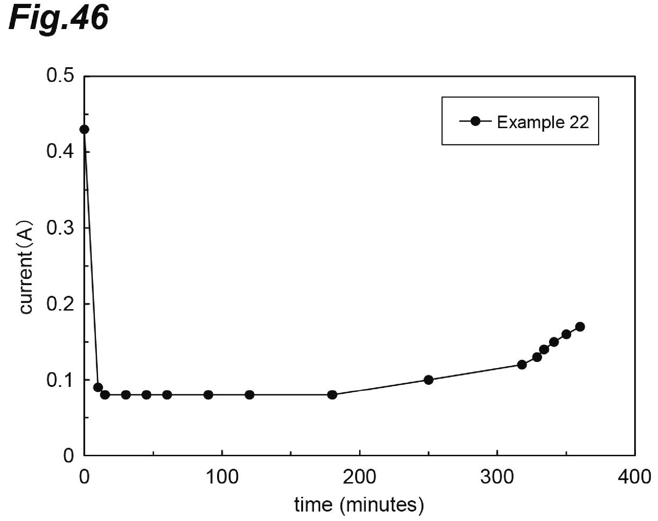

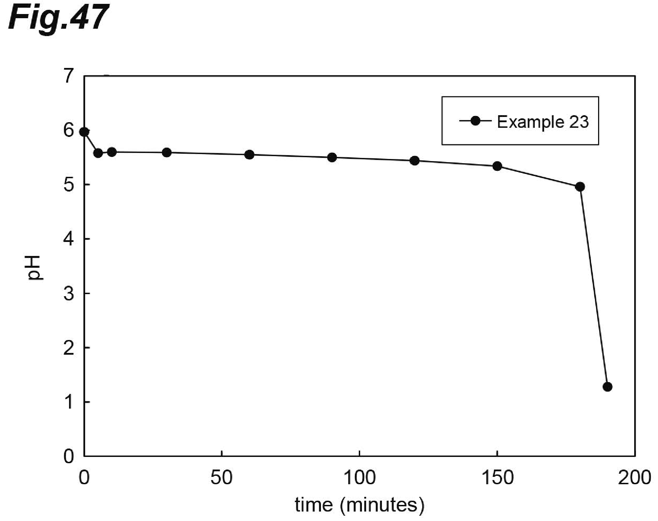

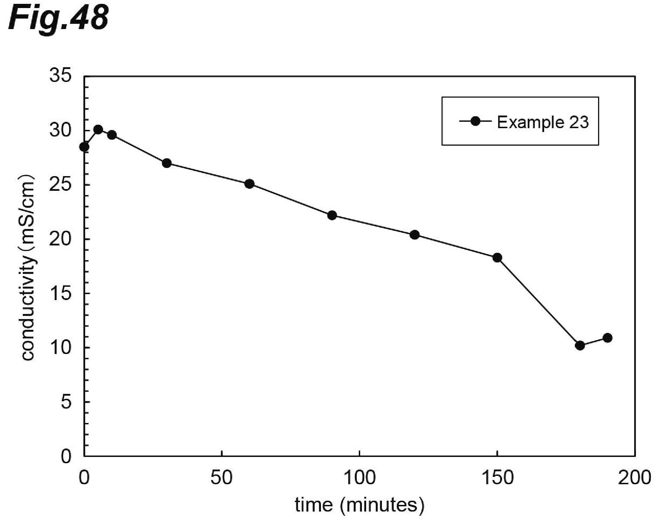

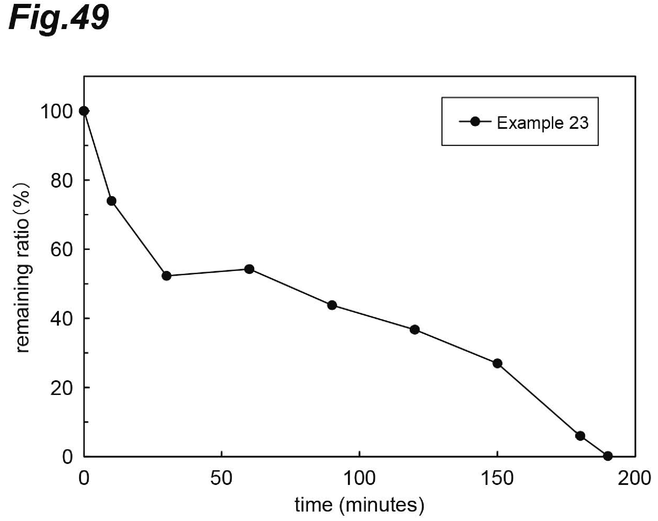

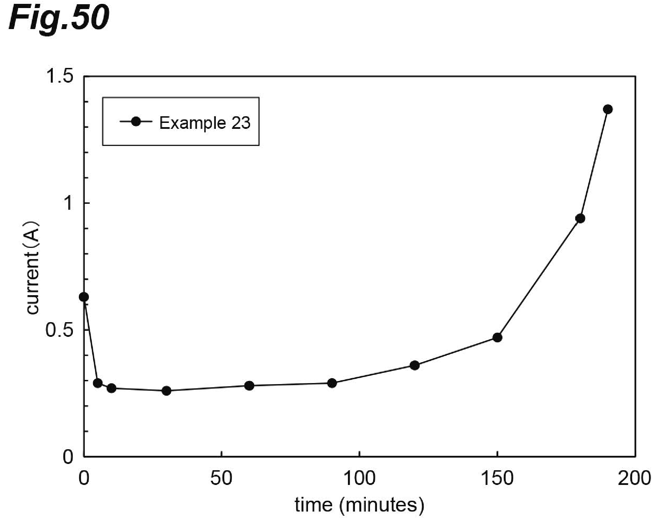

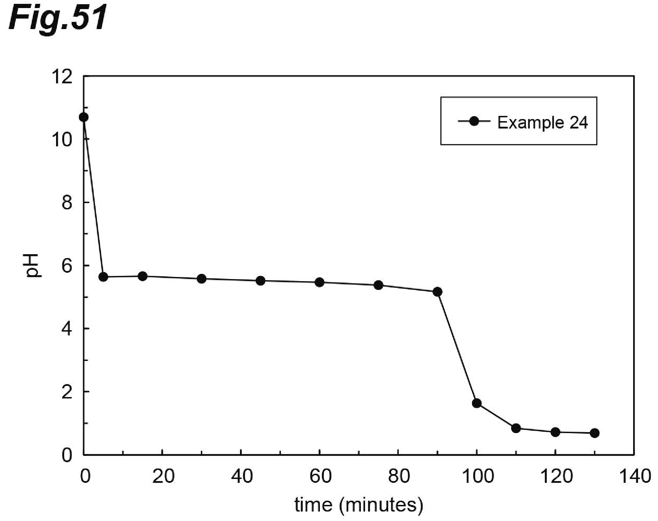

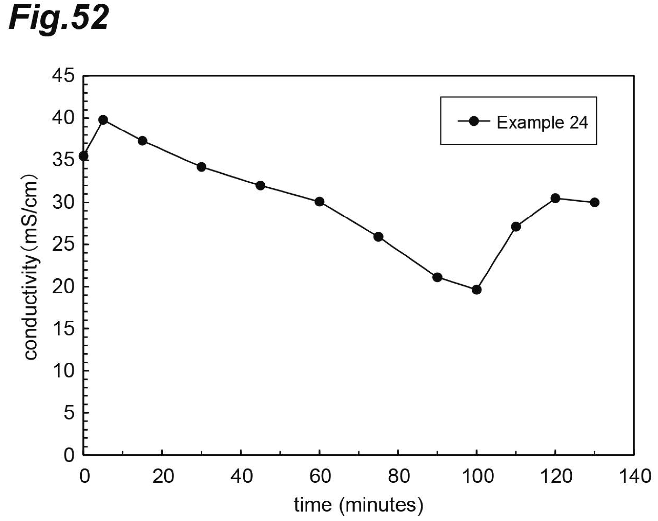

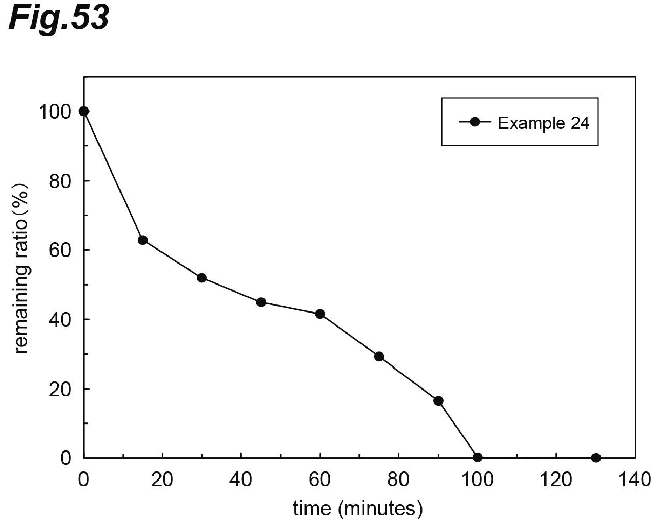

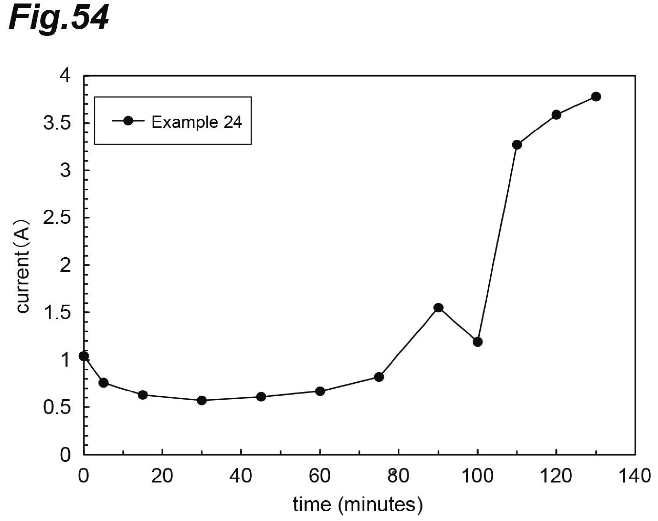

Table 16 shows conditions of electrodialysis tests in Examples 22, 23, and 24. In the electrodialysis tests of Examples 22, 23, and 24, no membrane clogging occurred, and crystals were precipitated in the desalination chamber 30. Before initiation of electrodialysis and during electrodialysis, the pH and conductivity of the liquid in the desalination chamber, a current value flowing through the electrodialysis apparatus, and a UV absorbance (240 nm) of a supernatant were measured. From a ratio of the UV absorbance (240 nm) at each measurement time after initiation of electrodialysis to the UV absorbance (240 nm) before initiation of electrodialysis, a concentration of terephthalate ions remaining in the supernatant was calculated. FIG. 43 is a graph showing a relationship between pH in the desalination chamber and energization time in Example 22. FIG. 44 is a graph showing a relationship between conductivity in the desalination chamber and energization time in Example 22. FIG. 45 is a graph showing a relationship between energization time and a remaining ratio of disodium terephthalate in Example 22. FIG. 46 is a graph showing a relationship between a current value flowing through the electrodialysis apparatus and energization time in Example 22. FIG. 47 is a graph showing a relationship between pH in the desalination chamber and energization time in Example 23. FIG. 48 is a graph showing a relationship between conductivity in the desalination chamber and energization time in Example 23. FIG. 49 is a graph showing a relationship between energization time and a remaining ratio of disodium terephthalate in Example 23. FIG. 50 is a graph showing a relationship between a current value flowing through the electrodialysis apparatus and energization time in Example 23. FIG. 51 is a graph showing a relationship between pH in the desalination chamber and energization time in Example 24. FIG. 52 is a graph showing a relationship between conductivity in the desalination chamber and energization time in Example 24. FIG. 53 is a graph showing a relationship between energization time and a remaining ratio of disodium terephthalate in Example 24. FIG. 54 is a graph showing a relationship between a current value flowing through the electrodialysis apparatus and energization time in Example 24. It was confirmed that disodium terephthalate contained in the depolymerization liquid obtained by alkaline depolymerization of PET can be recovered by crystallization as free terephthalic acid. The obtained free terephthalic acid can be used as a raw material for polyester.

[Table 16]

| Circulation Path | | Example 22 | Example 23 | Example 24 |
|---|---|---|---|---|
| C1 | NaOH concentration [M] | 0.5 | 0.5 | 0.5 |
| | Solution volume [mL] | 1000 | 1000 | 1000 |
| C2 | $H_2SO_4$ concentration [M] | 0.1 | 1 | 1 |
| | Solution volume [mL] | 500 | 500 | 500 |

(continued)

| Circulation Path | | Example 22 | Example 23 | Example 24 |
|---|---|---|---|---|
| C3 | $Na_2TPA$ concentration [M] | 0.1 | 0.4 | 0.54 |
| | NaOH concentration [M] | 0.019 | 0.076 | 0.1 |
| | $Na_2SO_4$ concentration [M] | 0.05 | 0.05 | 0.2 |
| | EG concentration [M] | 0.1 | 0.4 | 0.54 |
| | MeOH concentration [M] | 0.30 | 1.20 | 1.62 |
| | Solution volume [mL] | 200 | 100 | 100 |
| C4 | NaOH concentration [M] | 0.01 | 0.01 | 0.01 |
| | Solution volume [mL] | 500 | 500 | 500 |
| | Applied voltage [V] | 15 | 35 | 35 |

Example 25

Electrodialysis was performed under the same conditions as in Example 13, except that an amount of the $H_2SO_4$ aqueous solution circulated through the circulation path C2 was changed to 1000 mL. No membrane clogging occurred, and crystals were precipitated in the desalination chamber 30. From a ratio of the UV absorbance (240 nm) at each measurement time after initiation of electrodialysis to the UV absorbance (240 nm) before initiation of electrodialysis, a concentration of terephthalate ions remaining in the supernatant was calculated. At a point of 90 minutes of energization, terephthalate ions remaining in the supernatant decreased to about 10%, and 90% was converted into free terephthalic acid and precipitated as crystals. An aqueous solution containing crystals of terephthalic acid was recovered from the circulation path C3. A particle size distribution of the obtained terephthalic acid crystals was measured using a laser diffraction particle size distribution analyzer based on a scattering method. From the obtained particle size distribution, a 20% particle diameter (D20 median diameter), a 50% particle diameter (D50 median diameter), and an 80% particle diameter (D80 median diameter) in terms of a cumulative distribution were calculated. Results are shown in Table 17.

Comparative Example 2

After diluting 10 mL of a 0.4 M disodium terephthalate aqueous solution (containing 0.1 M NaOH) with 30 mL of water, a 1 M $H_2SO_4$ aqueous solution was added dropwise with stirring to crystallize free terephthalic acid. When the pH decreased to 2 or less, a particle size distribution of the obtained terephthalic acid crystals was measured in the same manner as in Example 25. Results are shown in Table 17.

[Table 17]

| | $D_{20}$ median diameter [μm] | $D_{50}$ median diameter [μm] | $D_{80}$ median diameter [μm] |
|---|---|---|---|
| Example 25 | 16.0 | 101 | 281 |
| Comparative Example 2 | 1.91 | 9.40 | 20.9 |

As shown in Table 17, it was confirmed that terephthalic acid crystals obtained by the method of the examples had a significantly larger size than crystals obtained by a general neutralization crystallization method.

**Reference Signs List**

2, 2a: bipolar membrane;
3a: first cation exchange membrane;
3b: second cation exchange membrane;
4: anion exchange membrane;
5: electrodialysis unit;
11: anode;

13: cathode;
15: electrolyte tank;
20: acid component chamber;
25: acid component chamber tank;
30: desalination chamber;
35: desalination chamber tank;
40: alkali component chamber;
45: alkali component chamber tank;
50: electrodialysis tank;
100, 101: recovery apparatus;
C1, C2, C3, C4: circulation paths.

## Claims

**1.** A method for producing an organic acid, comprising:

preparing an electrodialysis tank comprising an anode, a cathode, and one or more electrodialysis units provided between the anode and the cathode, the electrodialysis unit comprising, sequentially arranged from an anode side toward a cathode side, a bipolar membrane, a first cation exchange membrane, and a second cation exchange membrane, an acid component chamber being formed between the bipolar membrane and the first cation exchange membrane, a desalination chamber being formed between the first cation exchange membrane and the second cation exchange membrane, and an alkali component chamber being formed on the cathode side of the second cation exchange membrane; and

while applying a voltage between the anode and the cathode, circulating an acid aqueous solution through a circulation path comprising the acid component chamber, circulating an alkali aqueous solution through a circulation path including the alkali component chamber, and circulating an organic acid salt aqueous solution comprising an organic acid salt through a circulation path including the desalination chamber, thereby precipitating a solid containing a free organic acid generated from the organic acid salt in the desalination chamber.

**2.** The method according to claim 1, wherein the solid comprises a crystal of the free organic acid.

**3.** The method according to claim 2, wherein the crystal is a needle-shaped crystal.

**4.** The method according to claim 1, wherein the organic acid salt aqueous solution further comprises an inorganic salt.

**5.** The method according to claim 4, wherein the inorganic salt comprises one or more selected from the group consisting of sodium chloride, potassium chloride, sodium sulfate, potassium sulfate, sodium nitrate, and sodium dihydrogen phosphate.

**6.** The method according to claim 4 or 5, wherein a concentration of the inorganic salt in the organic acid salt aqueous solution at 25°C before application of the voltage between the anode and the cathode is 0.001 M or more and 1.0 M or less.

**7.** The method according to claim 1, wherein the acid aqueous solution comprises an acid, and a concentration of the acid in the acid aqueous solution at 25°C before application of the voltage between the anode and the cathode is 0.001 M or more and 2.0 M or less.

**8.** The method according to claim 1, wherein the organic acid salt is one or more selected from the group consisting of a carboxylate, a sulfonate, a sulfinate, an organic phosphate, and an organic phosphite.

**9.** The method according to claim 1, wherein the organic acid salt is a carboxylate.

**10.** The method according to claim 1, wherein the organic acid salt is a salt of a dicarboxylic acid.

**11.** The method according to claim 1, wherein the organic acid salt is a salt of terephthalic acid.

**12.** The method according to claim 1, further comprising recovering an alkali component from the alkali component

chamber.

Fig.1

100
5
5
5
11
2
3a
3b
2
3a
3b
2
2
3a
3b
2a
13
45
35
25
+
12
20
30
40
20
30
40
20
30
40
14
−
15
C2
C3
C4
50
C1

13
2a
$H^+$
50
$OH^-$
40
NaOH
3b
$Na^+$
30
XH
3a
$X^-$
5
20
2
$H^+$
$OH^-$
11

*Fig.2*

*Fig.3*

(a)

non-liquid-contact portion
liquid-contact portion
TM4000 10kV 10.3mm X30 Mix L
1.00mm

(b)

TM4000 10kV 10.4mm X500 Mix L
100μm

*Fig.4*

(a)

TM4000 10kV 10.4mm X500 Mix L
100μm

(b)

TM4000 10kV 10.4mm X1.50k Mix L
30.0μm

*Fig.5*

liquid-contact portion
non-liquid-contact portion
liquid-contact portion
- non-liquid-contact portion
terephthalic acid(free)
disodium terephthalate
Absorbance (a.u.)
4000
3500
3000
2500
2000
1500
1000
500
Wavenumber (cm-1)

# Fig.6

(a)

non-liquid-contact portion
TM4000 10kV 9.4mm X50 Mix M
1.00mm

(b)

TM4000 10kV 9.5mm X1.00k Mix M
50.0µm

# Fig.7

(a)

liquid-contact portion
TM4000 10kV 9.4mm X50 Mix M
1.00mm

(b)

TM4000 10kV 9.5mm X1.00k Mix M
50.0µm

# Fig.8

liquid-contact portion
non-liquid-contact portion
liquid-contact portion
- non-liquid-contact portion
terephthalic acid(free)
disodium terephthalate
Absorbance (a.u.)
4000
3500
3000
2500
2000
1500
1000
500
Wavenumber (cm-1)

Fig.9

14
12
10
8
6
4
2
0
pH
6
5
4
3
2
1
0
conductivity (mS/cm)
0
10
20
30
40
50
60
70
time (minutes)
pH
conductivity

# Fig.10

pH
conductivity
14
12
10
8
6
4
2
0
6
5
4
3
2
1
0
0
10
20
30
40
50
60
70
time (minutes)
conductivity (mS/cm)

# *Fig.11*

Example 1
Example 2
remaining ratio (%)
100
80
60
40
20
0
0
10
20
30
40
50
60
70
time (minutes)

# Fig.12

0.5
0.4
0.3
0.2
0.1
0
current (A)
0
10
20
30
40
50
60
70
time (minutes)
Example 1
Example 2

# Fig.13

20 μm
TM4000 10kV 9.2mm X1.50k Mix M
30.0μm

# *Fig.14*

14
12
10
8
6
4
2
0
pH
0
10
20
30
40
50
60
70
time (minutes)
0.001 M
0.005 M
0.01 M
0.02 M
0.05 M
0.1 M

# *Fig.15*

30
25
20
15
10
5
0
0
20
40
60
80
conductivity (mS/cm)
time (minutes)
0.001 M
0.005 M
0.01 M
0.02 M
0.05 M
0.1 M

# Fig.16

0.001 M
0.005 M
0.01 M
0.02 M
0.05 M
0.1 M
remaining ratio (%)
time (minutes)
0
20
40
60
80
100
0
10
20
30
40
50
60
70

*Fig.17*

0.7
0.6
0.5
0.4
0.3
0.2
0.1
0
current (A)
0
10
20
30
40
50
60
70
80
time (minutes)
0.001 M
0.005 M
0.01 M
0.02 M
0.05 M
0.1 M

# Fig.18

14
12
10
8
6
4
2
0
pH
0
20
40
60
80
100
time (minutes)
0.01 M
0.05 M
0.1 M

Fig.19

0.01 M
0.05 M
0.1 M
conductivity (mS/cm)
time (minutes)
0
2
4
6
8
10
12
14
0
20
40
60
80
100

Fig.20

0.01 M
0.05 M
0.1 M
remaining ratio (%)
100
80
60
40
20
0
0
20
40
60
80
100
time (minutes)

# Fig.21

0.5
0.4
0.3
0.2
0.1
0
current (A)
0
20
40
60
80
100
time (minutes)
0.01 M
0.05 M
0.1 M

Fig.22
101
15
13
50
C1
5
2
14
3
40
4
30
20
12
11
+
25
35
45
C2
C3
C4

*Fig.23*

14
12
10
8
6
4
2
0
pH
0
2
4
6
8
10
time (minutes)
Example 9
Example 10
Example 11

*Fig.24*

30
25
20
15
10
5
0
conductivity (mS/cm)
0
2
4
6
8
10
time (minutes)
Example 9
Example 10
Example 11

Fig.25

Example 9
Example 10
Example 11
remaining ratio (%)
100
80
60
40
20
0
0
2
4
6
8
10
time (minutes)

*Fig.26*

5
4
3
2
1
0
current (A)
0
2
4
6
8
10
time (minutes)
Example 9
Example 10
Example 11

*Fig.27*

14
12
10
8
6
4
2
0
pH
0
50
100
150
200
250
time (minutes)
Example 12
Example 13
Example 14

# Fig.28

50
40
30
20
10
0
conductivity (mS/cm)
0
50
100
150
200
250
time (minutes)
Example 12
Example 13
Example 14

# Fig.29

110
100
90
80
70
60
50
40
30
20
10
0
remaining ratio (%)
0
50
100
150
200
250
time (minutes)
Example 12
Example 13
Example 14

# *Fig.30*

Example 12
Example 13
Example 14
current (A)
time (minutes)
0
1
2
3
4
5
0
50
100
150
200
250

# Fig.31

14
12
10
8
6
4
2
0
pH
0
100
200
300
400
time (minutes)
Example 15
Example 16
Example 17

# Fig.32

40
30
20
10
0
conductivity (mS/cm)
0
100
200
300
400
time (minutes)
Example 15
Example 16
Example 17

# Fig.33

120
100
80
60
40
20
0
remaining ratio (%)
0
100
200
300
400
time (minutes)
Example 15
Example 16
Example 17

*Fig.34*

Example 15
Example 16
Example 17
current (A)
0.6
0.4
0.2
0
0
100
200
300
400
time (minutes)

*Fig.35*

14
12
10
8
6
4
2
0
pH
0
100
200
300
400
time (minutes)
Example 18
Example 16
Example 19

# Fig.36

40
30
20
10
0
conductivity (mS/cm)
0
100
200
300
400
time (minutes)
Example 18
Example 16
Example 19

# Fig.37

120
100
80
60
40
20
0
remaining ratio (%)
0
100
200
300
400
time (minutes)
Example 18
Example 16
Example 19

# Fig.38

current (A)
1
0.8
0.6
0.4
0.2
0
0
100
200
300
400
time (minutes)
Example 18
Example 16
Example 19

# Fig.39

14
12
10
8
6
4
2
0
pH
0
20
40
60
80
100
time (minutes)
Example 16
Example 20
Example 21

# Fig.40

Example 16
Example 20
Example 21
conductivity (mS/cm)
30
20
10
0
0
20
40
60
80
100
time (minutes)

# Fig.41

100
80
60
40
20
0
remaining ratio (%)
0
20
40
60
80
100
time (minutes)
Example 16
Example 20
Example 21

# Fig.42

3
2.5
2
1.5
1
0.5
0
current (A)
0
20
40
60
80
100
time (minutes)
Example 16
Example 20
Example 21

*Fig.43*

14
12
10
8
6
4
2
0
pH
0
100
200
300
400
time (minutes)
Example 22

# *Fig.44*

25
20
15
10
5
0
conductivity (mS/cm)
0
100
200
300
400
time (minutes)
Example 22

# Fig.45

Example 22
remaining ratio (%)
time (minutes)
0
20
40
60
80
100
0
100
200
300
400

# *Fig.46*

0.5
0.4
0.3
0.2
0.1
0
current (A)
0
100
200
300
400
time (minutes)
Example 22

***Fig.47***

Example 23
pH
0
1
2
3
4
5
6
7
0
50
100
150
200
time (minutes)

# Fig.48

35
30
25
20
15
10
5
0
conductivity (mS/cm)
0
50
100
150
200
time (minutes)
Example 23

***Fig.49***

Example 23
remaining ratio (%)
100
80
60
40
20
0
0
50
100
150
200
time (minutes)

# Fig.50

1.5
1
0.5
0
current (A)
Example 23
0
50
100
150
200
time (minutes)

# Fig.51

12
10
8
6
4
2
0
pH
0
20
40
60
80
100
120
140
time (minutes)
Example 24

# Fig.52

45
40
35
30
25
20
15
10
5
0
conductivity (mS/cm)
Example 24
0
20
40
60
80
100
120
140
time (minutes)

# *Fig.53*

Example 24
remaining ratio (%)
100
80
60
40
20
0
0
20
40
60
80
100
120
140
time (minutes)

# *Fig.54*

4
3.5
3
2.5
2
1.5
1
0.5
0
current (A)
Example 24
0
20
40
60
80
100
120
140
time (minutes)

## INTERNATIONAL SEARCH REPORT

International application No.
**PCT/JP2024/030314**

**A. CLASSIFICATION OF SUBJECT MATTER**

***C02F 1/469***(2023.01)i
FI: C02F1/469

According to International Patent Classification (IPC) or to both national classification and IPC

**B. FIELDS SEARCHED**

Minimum documentation searched (classification system followed by classification symbols)

C02F1/46-1/48; B01D53/22; B01D61/00-71/82; C02F1/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2005-333886 A (SHOWA DENKO K.K.) 08 December 2005 (2005-12-08) claims, examples, drawings | 1-12 |
| A | JP 2015-80744 A (MITSUBISHI CHEMICAL CORPORATION) 27 April 2015 (2015-04-27) claims, examples, drawings | 1-12 |
| A | JP 51-59073 A (IONICS, INCORPORATED) 22 May 1976 (1976-05-22) claims, drawings | 1-12 |
| A | JP 60-216884 A (TEIJIN ENGINEERING LTD.) 30 October 1985 (1985-10-30) claims, examples, drawings | 1-12 |
| A | JP 2002-507148 A (ELECTROSYNTHESIS COMPANY, INC.) 05 March 2002 (2002-03-05) claims, examples, drawings | 1-12 |
| A | JP 2016-191692 A (KURITA WATER IND LTD.) 10 November 2016 (2016-11-10) claims, drawings | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 October 2024** | **15 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/030314**

**C. DOCUMENTS CONSIDERED TO BE RELEVANT**

| Category* | Citation of document, with indication, where appropriate, of the relevant passages | Relevant to claim No. |
|---|---|---|
| A | JP 48-37919 B1 (ASAHI CHEMICAL INDUSTRY CO., LTD.) 14 November 1973 (1973-11-14) claims, drawings | 1-12 |
| A | JP 2008-214662 A (KURITA WATER IND LTD.) 18 September 2008 (2008-09-18) claims, drawings | 1-12 |
| A | JP 2010-269288 A (ASTOM KK) 02 December 2010 (2010-12-02) claims, drawings | 1-12 |

Form PCT/ISA/210 (second sheet) (July 2022)

**INTERNATIONAL SEARCH REPORT**
**Information on patent family members**

International application No.
**PCT/JP2024/030314**

| Patent document cited in search report | Publication date (day/month/year) | Patent family member(s) | Publication date (day/month/year) |
|---|---|---|---|
| JP 2005-333886 A | 08 December 2005 | (Family: none) | |
| JP 2015-80744 A | 27 April 2015 | (Family: none) | |
| JP 51-59073 A | 22 May 1976 | US 3964985 A<br>claims, figures<br>US 4057483 A<br>GB 1501276 A<br>DE 2547101 A1 | |
| JP 60-216884 A | 30 October 1985 | (Family: none) | |
| JP 2002-507148 A | 05 March 2002 | US 6004445 A<br>claims, figures<br>WO 1999/000178 A1<br>EP 1017481 A1 | |
| JP 2016-191692 A | 10 November 2016 | US 2018/0079663 A1<br>claims, figures<br>WO 2016/159051 A1<br>EP 3279900 A1 | |
| JP 48-37919 B1 | 14 November 1973 | (Family: none) | |
| JP 2008-214662 A | 18 September 2008 | WO 2008/105489 A1 | |
| JP 2010-269288 A | 02 December 2010 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H05271150 A

### Non-patent literature cited in the description

- *Separation and Purification Technology*, 2019, vol. 212, 929-940